# EUROPEAN PATENT APPLICATION

(11) **EP 1 767 198 A1**
(43) Date of publication of application: **28.03.2007**
(21) Application number: 05734737.9
(22) Date of filing: 19.04.2005
(51) Int. Cl.: A61K 31/19, A61K 31/20, A61K 31/22, A61K 31/23, A61P 1/04, A61P 1/14, A61P 3/00, A61P 3/02, A61P 3/04, A61P 5/08, A61P 9/00, A61P 17/02, A61P 19/02, A61P 19/10, A23L 1/30

(54) **REGULATOR OF PHYSIOLOGICAL FUNCTION OF GHRELIN AND USE THEREOF**

(30) Priority: 09.06.2004 JP 2004171245
(71) Applicant: KURUME UNIVERSITY, Kurume-shi, Fukuoka 830-0011 (JP); SUNTORY LIMITED, Osaka-shi, Osaka 530-8203 (JP)
(72) Inventor: KOJIMA, Masayasu, Kurume-shi, Fukuoka 8390801 (JP); Nishi, Yoshihiro, Kurume-shi, Fukuoka 8390861 (JP); KANGAWA, Kenji, Minoo-shi, Osaka 5620031 (JP); ABE, Keiichi Suntory Lim.,World Head Quaters, Tokyo 135-8631 (JP); IZUMI, Reiko Suntory Lim.,Research Center, Osaka 618-8503 (JP); NAKAMURA, Junichi Suntory Lim.,World Head Quaters, Tokyo 135-8631 (JP)
(74) Representative: Vossius & Partner
(86) International application number: PCT/JP2005/007465
(87) International publication number: WO 2005/120485

(57) **Abstract**

A regulator for regulating physiological functions, such as activity of increasing an intracellular calcium ion concentration, activity of promoting growth hormone secretion, activity of promoting eating, regulatory activity relating to fat accumulation, activity of ameliorating heart function and activity of stimulating gastric acid secretion, of ghrelin, which regulator comprises a fatty acid of carbon number 2-35 or its derivative, and use thereof.

## Description

### TECHNICAL FIELD

The present invention relates to a regulator of physiological functions of ghrelin and use thereof in the field of medicines, foods or the like. Also, the present invention relates to an accelerator for the formation of modified ghrelins and foods comprising the same.

### BACKGROUND ART

Ghrelin is an intrinsic ligand (peptide hormone) for the receptor (GHS-R) which binds to a growth hormone secretagogue (GHS) being a synthetic and unnatural (non-natural) substance accelerating secretion of growth hormones, and which has been identified for the first time by Kojima, Kangawa et al. who are co-inventors of the present invention [Document 1 of documents listed in the "LIST OF REFERENCES" described below (hereinafter referred to as "D") and WO 01/007475]. At first, ghrelin was purified from the rat stomach, whereas it has been reported that ghrelin is expressed also in the brain, lung, kidney, pancreas, small intestine, and large intestine [D2-D7]. In addition, ghrelin has been isolated from or estimated to be present in a vertebrate animal other than rat, for example, human, mouse, swine, chicken, eel, cattle, horse, sheep, frog, rainbow trout or dog (JP 2004-2378 A). Ghrelin has activity of increasing an intracellular calcium ion concentration and a potent activity of promoting growth hormone secretion [D1, D8-D10]. In addition to these activities, ghrelin has a variety of activities such as activity of stimulating appetite, activity of inducing adiposity [D11-D14], activity of ameliorating cardiac functions [D15-D17], activity of stimulating gastric acid secretion [D18], and the like. Because ghrelin has a wide variety of physiological functions, the regulation of its functions should be significant not only for subjects suffering from diseases associated with ghrelin, but also for healthy subjects.

Ghrelins having been identified so far are a group of peptides consisting of about 30 or less amino acid residues, and have a structural feature that the position-3 (third) amino acid is substituted with an acyl group. For example, the human ghrelin is composed of 28 amino acids, and the third serine side chain is acylated with a fatty acid (*n*-octanoic acid, carbon number (C) is 8). The acylation of position-3 amino acid is known to be essential for expression of physiological activities of ghrelin such as activity of increasing intracellular calcium ion concentration, activity of promoting growth hormone secretion, and the like [D1]. Although ghrelin molecules normally contain serine at position 3 (hereinafter, they are expressed as "Ser³" or "ser(3)"), some ghrelin molecules contain different amino acid residue at position 3 ; for example, bullfrog ghrelin contains threonine (JP 2004-2378 A).

The acyl group to be utilized for the modification of the position-3 amino acid, which is essential for biological activities of ghrelin, is primarily a medium- to long-chain fatty acid residue. Ghrelins in mammals such as human, swine, cattle, sheep, dog, rat, mouse and the like, in birds such as chicken and the like, in fishes such as eel, rainbow trout, tilapia, catfish and the like as well as in amphibians such as frog and the like, are modified with an *n*-octanoyl group [D1, D19, and JP 2004-2378 A], whereas there is a small population of ghrelin peptides showing acyl-modifications of different type. Examples of such acyl-modifications include those wherein the acyl is *n*-decanoyl (C10:0, no double bonds, e.g., bullfrog shown in JP 2004-2378 A) or *n*-decenoyl (C10:1, one unsaturated bond, [D20-D22]). Other examples include modifications with *n*-butanoyl (C4, e.g., horse), hexanoyl (C6), dodecanoyl (C12), and the like (JP 2004-2378 A).

The acyl-modification of ghrelin is the first example of lipid-modification of peptide hormones. The modification of mammalian protein wherein the serine hydroxy group is acylated has not been reported either. There exist in the living body an acylated ghrelin (hereinafter, it may be referred to as "modified ghrelin") and a non-acylated ghrelin (hereinafter, it may be referred to as "unmodified ghrelin"). However, a putative enzyme catalyzing the transfer of an acyl group to the position-3 amino acid residue of ghrelin is likely a novel acyltransferase which is considered to be important in the regulation of ghrelin production. Such an enzyme has not been identified yet.

Applications of ghrelin in various fields such as medical field, stock farming, food industry, and the like, have been attempted with a focus on the potent physiological activities of ghrelin. Specifically, there has been proposed its use as an agent for treating eating disorder, an agent for promoting growth hormone secretion, and the like [WO 01/007475, JP 2004-2378 A and WO 2002/060472]. These applications are predicated on the usage of a synthetic ghrelin derivative or an analog. However, there are problems that, in the case where unmodified ghrelin should be used, it must be converted into an acylated ghrelin, and, in the case where modified ghrelin should be used, an effective method for producing acylated ghrelin must be established.

Accordingly, for an effective use of the physiological activities in a wide variety of fields such as medicines, veterinary medicines, stock farming and the like, the development of a reliable and effective method for regulating the physiological activities of ghrelin has been demanded.

For example, a substance regulating the acylation of the position-3 amino acid of ghrelin in the living body functions as "a regulator" or "a modulator" for the physiological functions (activities) of ghrelin, and is expected to be useful for increasing or suppressing a variety of physiological activities of ghrelin. Such a regulator can be used in the production of a pharmaceutical composition for treating or preventing a variety of physiological disorders associated with the physiological activities of ghrelin. Specific examples include a pharmaceutical composition for treating diseases caused by loss or decrease, or excess of a growth hormone. In addition, the pharmaceutical composition can be used for treating animals suffering from the conditions of anorexia and malnutrition as well as, in contrast, for treating animals exhibiting symptoms related to treatment of impaired health, obesity and the like associated with excessive appetite. Alternatively, the pharmaceutical composition is useful for accelerating fattening/growth of livestock or for reducing fatty meat.

As functional foods in the forms that can be taken routinely, there are commercially available drinkable preparation (Momentum Inc.; trade name "PM Formula") as well as dietary supplements (ForMor International; trade name "HGH Boost", Pure Supplements Products; "Height Assistance Supplements") and the like, in which an amino acid having activity of promoting growth hormone secretion, such as L-arginine or the like, is compounded. However, there have not been known at all a functional food that can regulate the acylation of the position-3 amino acid of ghrelin in the living body and a food that accelerates formation of modified ghrelin.

Also, there is a problem that the above-mentioned amino acid having activity of promoting growth hormone secretion, when used as a single material, does not promote the secretion of growth hormones unless a considerable amount is taken. Although a method has been proposed that promotes the secretion of growth hormones by compounding various kinds of amino acids, herbs, minerals and vitamins at a particular ratio (JP 2004-256513 A), its effect is not sufficient.

Furthermore, the functional foods are usually enriched with more than one kinds of components from vitamins, minerals, proteins, peptides, amino acids, lipids, hydrocarbons and the like. However, many components, except particular proteins, lipids, hydrocarbons and the like, have been used without elucidating their physiological functions, and, therefore, evaluation of their use is variable.

A drip or a fluid diet to be used during treatment usually contains merely the minimum nutrient components, and is not always effective for an aggressive improvement of the body functions. Accordingly, for a rapid and effective improvement of the body functions, there has been desired development of a drip, a fluid diet and the like having higher functions. Thus, a modulator of the physiological activities of ghrelin is considered to be extremely useful in a variety of applications for the above-mentioned functional foods, drips, fluid diets, livestock feeds and the like.

### DISCLOSURE OF THE INVENTION

An object of the present invention is to provide a substance regulating the acylation of the position-3 amino acid of ghrelin in the living body, and a method for controlling the physiological functions of ghrelin by the use of said substance.

Another object of the present invention is to provide a method for increasing or decreasing the concentration of modified ghrelin.

Still another object of the present invention is to provide a pharmaceutical composition or a food that exhibits a therapeutic effect and a health promotion effect through regulation of the physiological activities of ghrelin.

Other objects or effects of the present invention will be easily understood from the present specification and drawings.

As a result of investigations into a variety of synthetic, acyl-modified ghrelin peptides, the present inventors had found that the effects of biological activities of ghrelin can be altered by changing the acyl molecule [D23]. The present inventors have found that ingested (extrinsic) fatty acids are directly used for the acylation of the position-3 amino acid of ghrelin (for example, Ser(3)) in the living body, and that extrinsic fatty acids themselves are useful for the control of the physiological functions of ghrelin. In particular, the present inventors found that the concentration of modified ghrelin (an activated ghrelin) can be increased through the ingestion of a medium-chain fatty acid of 6 to 12 carbon number ("C₆ - C₁₂ fatty acid") as the extrinsic fatty acid, thereby resulting in completion of the present invention.

Thus, the present invention is related to the followings and the like.
(1) A regulator for regulating physiological functions of ghrelin, which comprises a fatty acid of carbon number 2 to 35 or its derivative.
(2) The regulator according to (1), wherein the physiological function of ghrelin is activity of increasing intracellular calcium ion concentration, activity of promoting growth hormone secretion, activity of promoting eating, regulatory activity relating to fat accumulation, activity of ameliorating cardiac function or activity of stimulating gastric acid secretion.
(3) A pharmaceutical composition comprising a regulator set forth in (1) or (2) above.
(4) A functional food comprising a regulator set forth in (1) or (2) above.
(5) An accelerator for the formation of activated ghrelin, which comprises at least one medium-chain fatty acid of 6 to 12 carbon number or its derivative.
(6) The accelerator for the formation of activated ghrelin according to (5) above, which comprises at least one medium-chain fatty acid of 8 to 10 carbon number or its derivative.
(7) A functional food containing an accelerator for the formation of activated ghrelin set forth in (5) or (6).
(8) A composition characterized in that it contains a medium-chain fatty acid of 6 to 12 carbon number or its derivative and that it has muscle-strengthening activity.
(9) A composition comprising a medium-chain fatty acid of 6 to 12 carbon number or its derivative which has skin-beautification activity.
(10) A method for preventing or treating disorders associated with physiological functions of ghrelin, which comprises administering an effective amount of a regulator set forth in (1) above, a pharmaceutical composition set forth in (3) above or a functional food set forth in (4) above to a subject in need thereof.

The regulator of the present invention affects the acylation of the position-3 amino acid of intrinsic ghrelin and thereby increasing or decreasing the ratio of the modified ghrelin, and is effective for treatment or prevention of a variety of physiological disorders associated with physiological functions of ghrelin, particularly for treatment of diseases caused by loss or decrease, or excess of growth hormone as well as for treatment of anorexia, malnutrition and the like. Also, the regulator of the present invention is useful for improving, for example, the growth of livestock. Furthermore, the regulator of the present invention can also contribute to elucidation of the mechanism of acyl-modification of a peptide hormone, ghrelin, particularly to characterization of putative ghrelin Ser *O*-acyltransferase.

An accelerator for the formation of activated ghrelin that increases the ratio of modified ghrelin having physiological activity (also referred to as "an activated ghrelin"), among modified ghrelins, can enhance, for example, activities of ghrelin such as activity of increasing intracellular calcium ion concentration or activity of promoting growth hormone secretion, and whereby exhibits physiological effects such as strengthening of muscles/skeletons, decrease of fats, rejuvenescence of skin, refreshment, and the like. The accelerator for the formation of activated ghrelin of the present invention being safe and free of side effects, said accelerator can be used in the form of a food, which makes it possible to be taken routinely. As a result, efficient expression of above-mentioned physiological effects can be achieved.

### BRIEF DESCRIPTION OF DRAWINGS

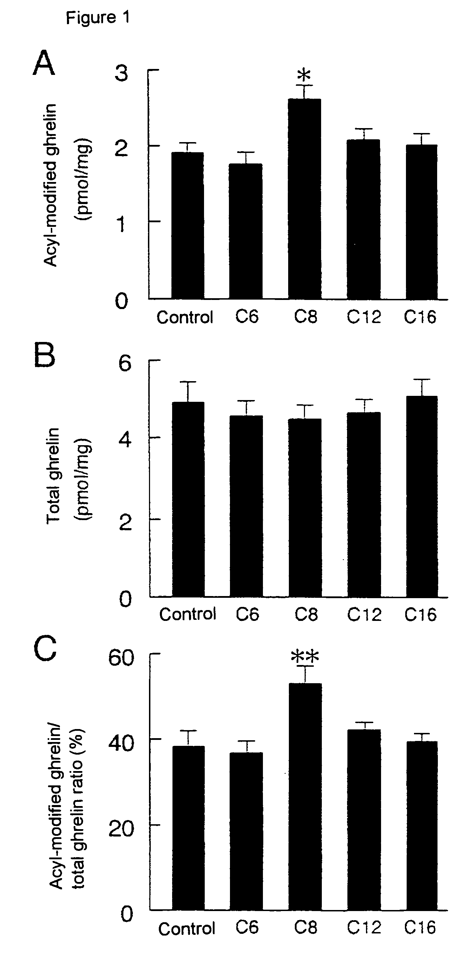

Figure 1 shows ghrelin concentrations in the stomach of mice fed *n*-hexanoic acid (C6), *n*-octanoic acid (C8), *n*-lauric acid (C 12), or *n-*palmitic acid (C16) and normal control animals (Control) fed standard chow and water. A, Acyl-modified ghrelin concentrations measured by ghrelin N-RIA (n=8). As N-RIA is very specific for acyl-modified ghrelin and the main form of acylated ghrelin is *n*-octanoyl ghrelin, the acyl-modified ghrelin concentration measured by N-RIA primarily reflects *n-*octanoyl ghrelin population. B, Total ghrelin concentration measured by ghrelin C-RIA (n=8). The total ghrelin concentration includes both acylated and des-acyl ghrelin. C, The ratio of acyl-modified/total ghrelin. Data represent mean ± S.D. of ghrelin concentrations in stomach extracts (from 1 mg wet weight). Asterisks indicate statistical significance. *, p<0.01; **, p<0.001 vs. control.

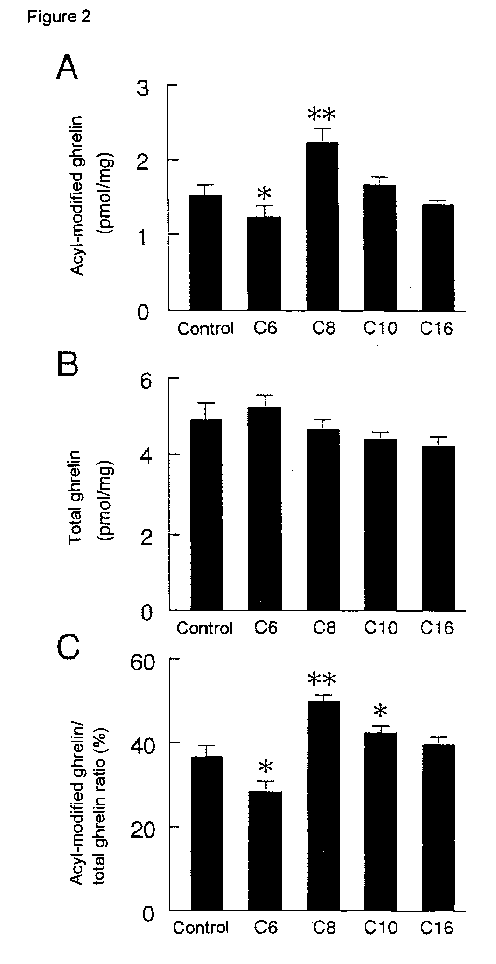

Figure 2 shows ghrelin concentration in the stomach of mice fed chow mixed with glyceryl trihexanoate (C6), glyceryl trioctanoate (C8), glyceryl tridecanoate (C10), or glyceryl tripalmitate (C16) and in that from mice fed standard laboratory chow (Control) (n=8). A, Acyl-modified ghrelin concentrations measured by ghrelin N-RIA. B, Total ghrelin concentration measured by ghrelin C-RIA. Data represent mean ± S.D. of ghrelin concentration in stomach extracts (from 1mg wet weight) (n=5). C, The ratio of acyl-modified/total ghrelin concentration. Data represent mean ± S.D. of calculated ratios (n=5). Asterisks indicate statistical significance. *, p<0.05; **, p<0.01 vs. control.

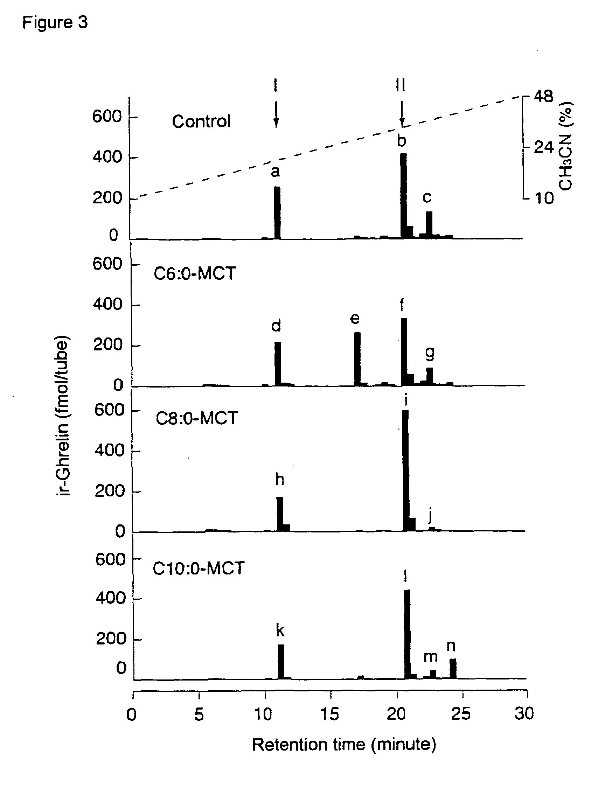

Figure 3 shows the molecular forms of ghrelin peptides from the stomachs of mice fed chow containing glyceryl trihexanoate (C6:0-MCT), glyceryl trioctanoate (C8:0-MCT), glyceryl tridecanoate (C10:0-MCT), and standard laboratory chow (Control). Peptide extracts from mouse stomachs were fractionated by HPLC and measured for ghrelin immunoreactivities by C-RIA. An assay tube contained equivalent quantities of peptide extract derived from 0.2 mg of stomach tissue. The black bars represent immunoreactive ghrelin (ir-ghrelin) concentrations measured by ghrelin C-RIA. Arrows indicate the elution positions of des-acyl ghrelin (I) and *n*-octanoyl ghrelin (II). Based on the retention times of synthetic ghrelin, peaks a, d, h, and k corresponded to those of des-acyl ghrelin, and peaks b, f, i, and 1 corresponded to those of *n*-octanoyl ghrelin, peaks c, g, j, and m corresponded to those of *n*-decenoyl (C10:1) ghrelin and peaks n corresponded to that of *n*-decanoyl (C10:0) ghrelin.

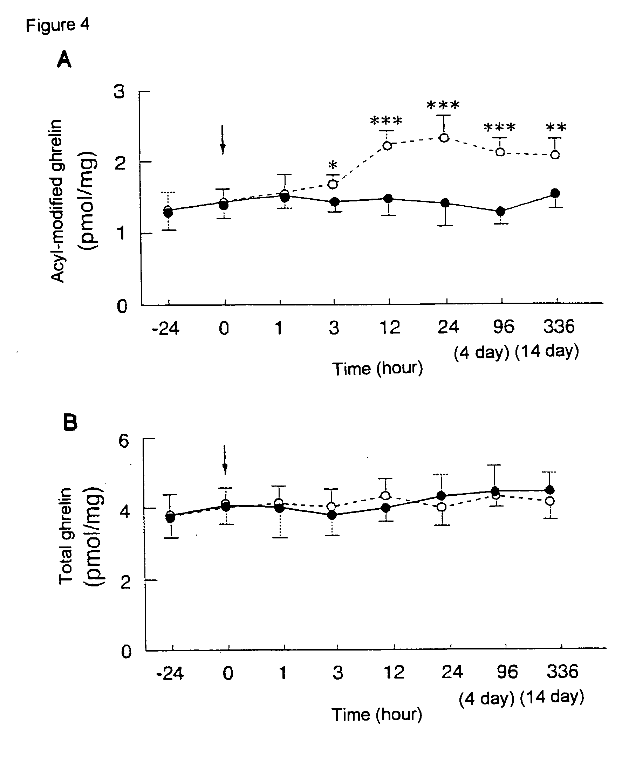

Figure 4 shows time-dependent changes in the stomach concentrations of ghrelin in mice fed glycerol trioctanoate. A, Acyl-modified ghrelin content measured by ghrelin N-RIA. B, Total ghrelin content measured by ghrelin C-RIA. After 12 hours of fasting, glyceryl trioctanoate (5% w/w)-containing chow was given to mice beginning at the time (0 hr) indicated by the arrow. Stomach samples were isolated from control mice fed standard laboratory chow (closed circles) and mice fed glyceryl trioctanoate (open circles) at the indicated times. Each point represents mean ± S.D. (n = 8). Asterisks indicate statistical significance. *, p<0.05; **, p<0.01 and ***, p<0.001 vs. control.

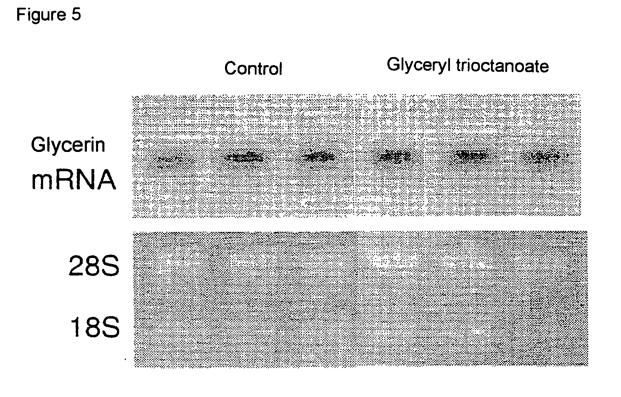

Figure 5 shows Northern blot analysis examining stomach ghrelin mRNA expression after ingestion of glyceryl trioctanoate-containing chow. Each lane contained 2 µg of total RNA. The lower panel indicates 28S and 18S ribosomal RNAs internal controls.

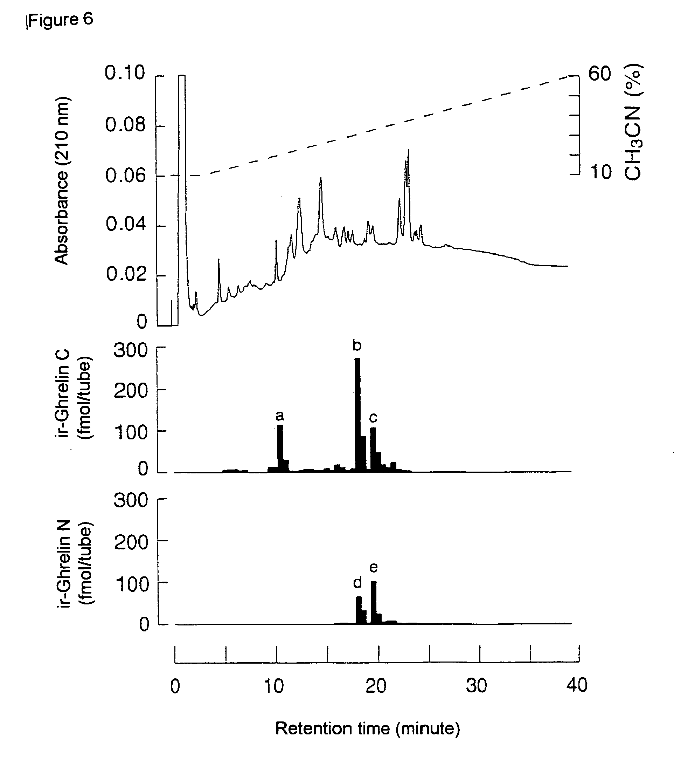

Figure 6 shows the HPLC profile of stomach extracts from mice fed glyceryl triheptanoate. Stomach extracts of glyceryl triheptanoate-treated mice were fractionated by HPLC (upper panel). The concentration of ghrelin in each fraction (from 0.2 mg stomach tissue equivalent) was monitored by C-RIA (middle panel) and N-RIA (lower panel). Ghrelin immunoreactivities, represented by solid bars, were separated into three major peaks (peaks a, b, and c) by C-RIA (middle panel) and two major peaks (peaks d and e) by N-RIA. Peaks b and d were observed only after ingestion of glyceryl triheptanoate.

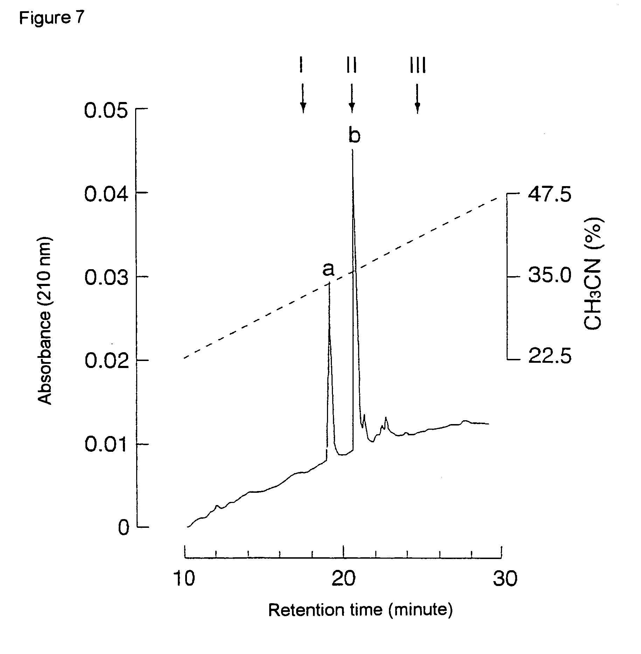

Figure 7 shows the final purification of *n*-heptanoyl ghrelin. Ghrelin peptides were purified from the stomachs of mice fed glyceryl triheptanoate. The sample eluted from an anti-rat ghrelin immunoaffinity column was subjected to HPLC. Peak a was observed only in samples from glyceryl triheptanoate-treated mice. Based on the retention times of HPLC and MALDI-TOF-MS analysis, peak b corresponded to *n*-octanoyl ghrelin. Arrows indicated the positions of elution of *n*-hexanoyl (I), *n*-octanoyl (II), and *n*-decanoyl (III) ghrelin, respectively.

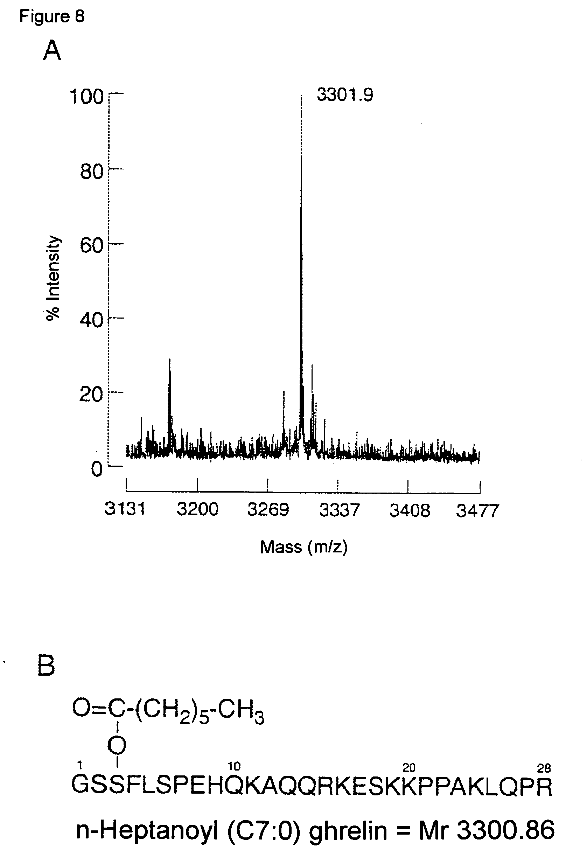

Figure 8 shows a matrix-assisted laser desorption/ionization time-of-flight mass spectrum of purified ghrelin-like peptide from Fig. 7 peak a. The mass ranges from 3131.0 to 3477.0 (m/z). From the averaged 100 mass spectra acquired in positive ion mode (average [M+H]+ : 3301.9), the molecular weight of the peak a peptide was calculated to be 3300.9. B, Structure of *n*-heptanoyl (C7:0) ghrelin. The calculated molecular weight of *n*-heptanoyl ghrelin is 3300.86.

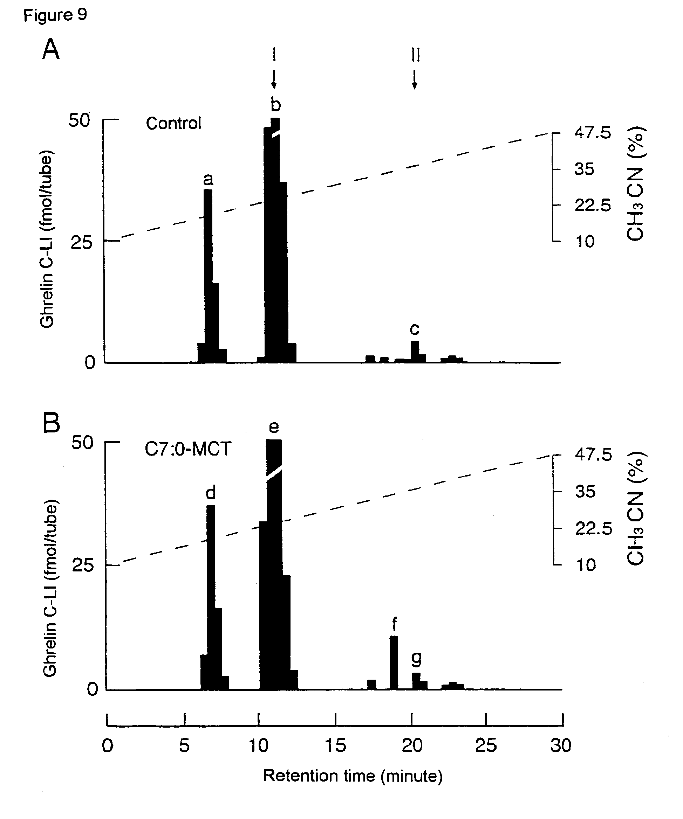

Figure 9 shows molecular forms of plasma ghrelin peptides from mice fed glyceryl triheptanoate-mixed chow. Plasma samples of control mice fed standard chow (A) and glyceryl triheptanoate-treated mice (B) were fractionated by HPLC and measured for ghrelin immunoreactivity by C-RIA. Arrows indicate the elution positions of des-acyl ghrelin (I) and *n*-octanoyl ghrelin (II). Solid bars represented plasma ghrelin immunoreactivities. Peaks b and e corresponded to those of des-octanoyl ghrelin, Peaks c and g corresponded to *n-*octanoyl ghrelin. Newly appeared Peak f represented the same retention time with that of *n*-heptanoyl ghrelin observed in the stomach of mice after glyceryl triheptanoate-treatment.

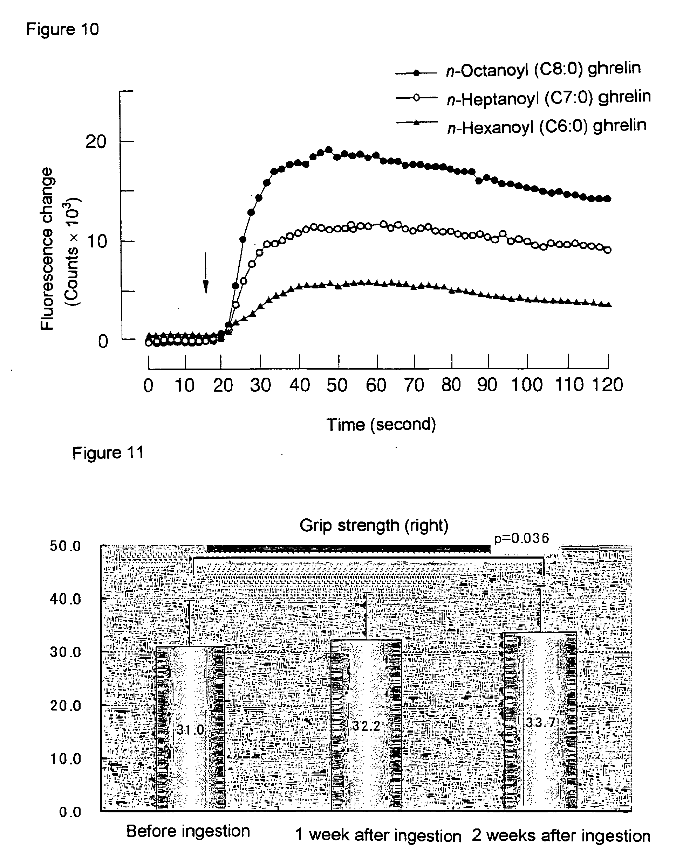

Figure 10 shows the time courses of fluorescence changes induced by *n*-octanoyl ghrelin (closed circle), *n*-heptanoyl ghrelin (open circle), and *n*-hexanoyl ghrelin (closed triangle) in GHS-R-expressing cells. Peptides (1 x 10⁻⁸ M) were added at the time indicated by the arrow.
Figure 11 shows changes in the grip strength of the dominant hand (right hand) following ingestion of a medium-chain fatty acid triglyceride.

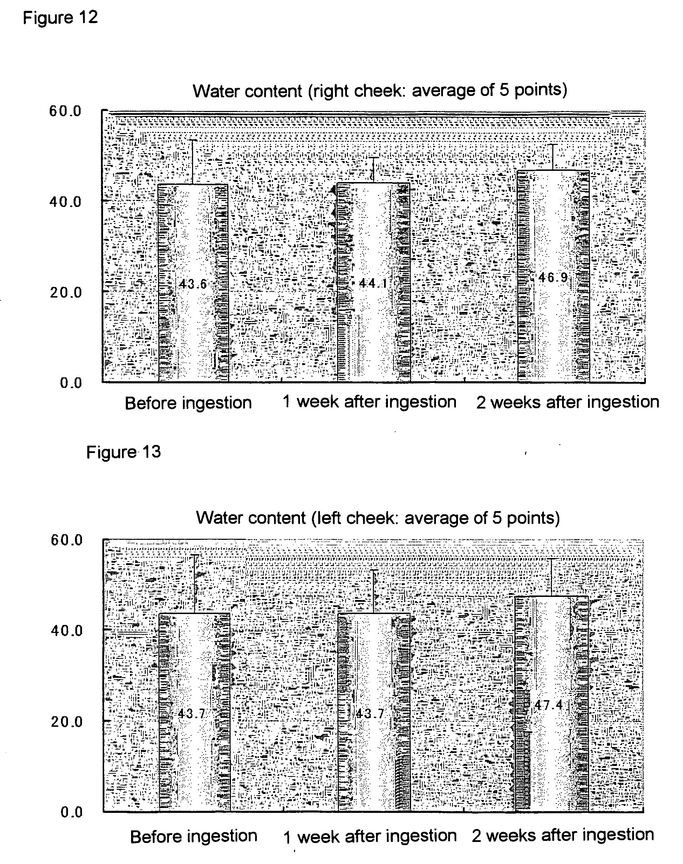

Figure 12 shows changes in the water content of facial skin (right cheek) following ingestion of a medium-chain fatty acid triglyceride.
Figure 13 shows changes in the water content of facial skin (left cheek) following ingestion of a medium-chain fatty acid triglyceride.

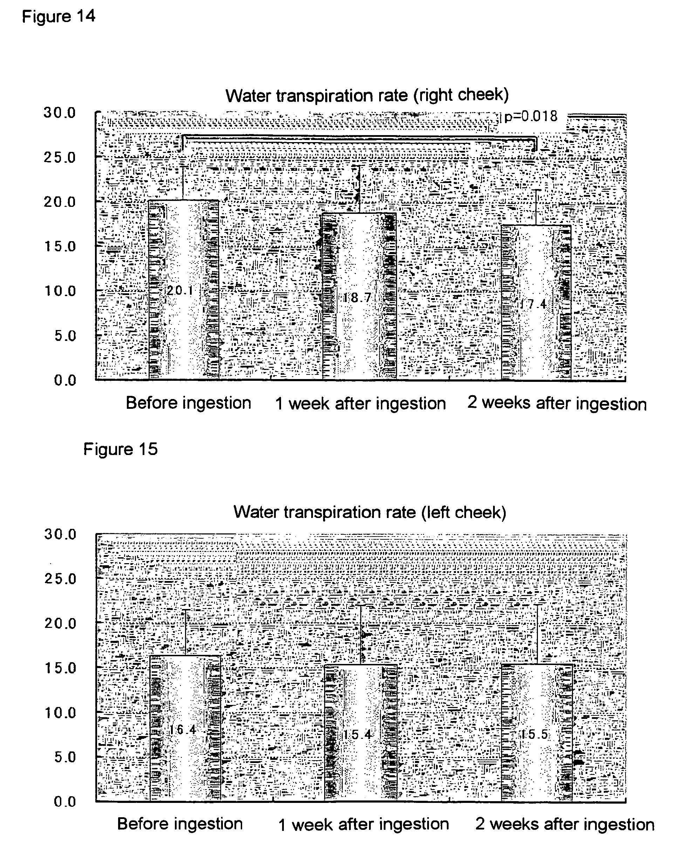

Figure 14 shows changes in the water transpiration rate of (amount of water transpiring from) facial skin (right cheek) following ingestion of a medium-chain fatty acid triglyceride.
Figure 15 shows changes in the water transpiration rate of facial skin (left cheek) following ingestion of a medium-chain fatty acid triglyceride.

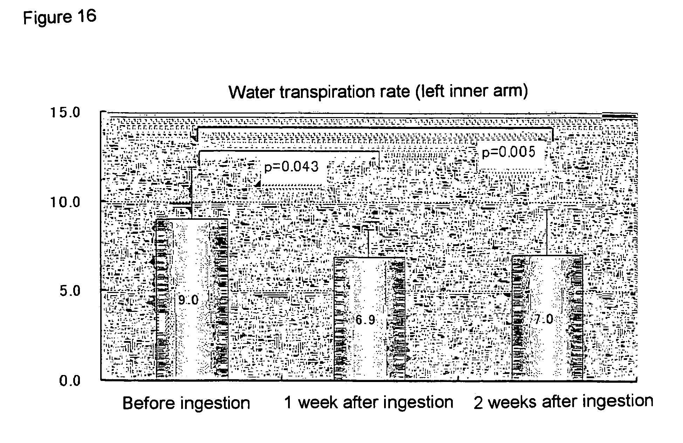

Figure 16 shows changes in the water transpiration rate of skin of left inner arm following the ingestion of a medium-chain fatty acid triglyceride.

### BEST MODE FOR CARRYING OUT THE INVENTION

The terms used throughout the specification and claims are described below

"Ghrelin" is a peptide hormone composed of about 30 amino acid residues, which binds to the receptor (GHS-R) of an intrinsic growth hormone secretagogue (GHS), and exhibits activity of increasing intracellular calcium ion concentration and also activity of promoting growth hormone secretion. Ghrelin is widely distributed in vertebrate animals, and has been identified in mammals, birds, fishes, amphibians and the like. Accordingly, the present invention encompasses ghrelins originating in arbitrary origins.

The preferred origins of ghrelins are livestock, poultry, pet fishes and the like, besides humans, such as swine, cattle, horse, sheep, rabbit, rat, mouse, dog, chicken, eel, rainbow trout, bullfrog and the like. Several kinds of ghrelins originating from them have been already isolated, and their amino acid sequences are known. See for example, JP 2004-2378 A.

In the present specification and claims, the term "(acyl)-modified ghrelin" means a peptide, in which the position-3 amino acid residue (for example, serine) of the ghrelin molecule is modified with an acyl group, and is simply referred to as "an acyl ghrelin".

The term "acylation" means substitution at the hydroxy group in the side chain of position-3 amino acid with an acyl group, preferably a fatty acid residue. Also, the term "unmodified ghrelin" means a peptide, in which the position-3 amino acid is not acylated, and is simply referred to as "des-acyl ghrelin". Furthermore, as stated above, modified ghrelin that exhibits physiological activities of ghrelin is referred to as "activated ghrelin ".

The term "regulator" for a physiological function(s) of ghrelin means a substance that, when administered to a living body expressing GHS-R of which ligand is ghrelin, enhances or weakens physiological functions of ghrelin. A substance that enhances physiological functions of ghrelin can be exemplified by a fatty acid with activating effect, which fatty acid has an acyl group that makes ghrelin physiologically active through acylation of the position-3 amino acid of ghrelin. On the other hand, a substance that weakens physiological functions of ghrelin can be exemplified by a fatty acid, which does not affect at all or rather lowers physiological functions of ghrelin, and acylates the position-3 amino acid of ghrelin in competition with the above-mentioned fatty acid having an activating effect.

As described in the examples below, in the case of mice, the ingestion of either of a medium-chain fatty acid (MCFA) or a medium-chain triacylglycerol (also referred to as "a medium-chain triglyceride") (MCT) increased the production of acyl-modified ghrelin without changing the concentration of the total ghrelins (acyl ghrelins and des-acyl ghrelins). In the case where either of MCFA or MCT was given to mice, the carbon-chain length of the acyl group bound to unmodified (initial) ghrelin corresponded to the carbon-chain length of the ingested MCFA or MCT. In contrast, a ghrelin peptide that was modified by *n-*butyryl group or *n*-palmitoyl group was not detected after the ingestion of the corresponding short-chain fatty acid (SCFA) or long-chain fatty acid (LCFA). Moreover, *n*-heptanoyl ghrelin (ghrelin in unnatural form) was produced in the stomachs of mice after the ingestion of *n*-heptanoic acid or glyceryl triheptanoate. These findings indicate that fatty acids utilized in the acylation of ghrelin have a certain carbon chain, that the ingested fatty acids are directly utilized in the acyl modification of ghrelin, and that a putative enzyme catalyzing the acyl-modification of ghrelin possibly has higher affinity to such certain fatty acids. In the case of human ghrelin or mouse ghrelin that is predominantly acylated by a medium-chain fatty acid, such an enzyme has higher affinity to MCFA than to SCFA or LCFA.

In the case of mice where ghrelin is acylated by medium-chain fatty acids, ingestion of medium-chain fatty acids (*n*-hexanoic acid, *n-*octanoic acid and *n*-decanoic acid) or medium-chain triglycerides (glyceryl trihexanoate, glyceryl trioctanoate and glyceryl tridecanoate) increased the gastric concentrations of ghrelins modified by acyl groups having carbon chain of corresponding lengths (namely, *n*-hexanoyl ghrelin, *n*-octanoyl ghrelin and *n*-decanoyl ghrelin). Also, ingestion of glyceryl triheptanoate (cannot be synthesized by mammalian cells) resulted in the production of ghrelin in unnatural form that is modified by *n*-heptanoyl. However, ingestion of fatty acid did not increase the total production of ghrelins (acyl-modified and des-acyl ghrelins) significantly. These findings indicate that the ingested medium-chain fatty acids and medium-chain triglycerides are the direct lipid sources for the acyl-modification of ghrelin.

Thus, fatty acids and triglycerides, when ingested, are utilized as lipid sources in the acyl-modification of ghrelin, and, in this way, affect the concentrations of acyl-modified ghrelins. This means that they function as a regulator for physiological functions of ghrelin. Specifically, a fatty acid that binds to the position-3 amino acid of ghrelin to increase the physiological functions of ghrelin is "a positive regulator". On the other hand, a fatty acid that does not affect or inhibits the physiological functions of ghrelin is "a negative regulator".

The present invention will be described by exemplifying mainly ghrelin having serine as the position-3 amino acid though, a person skilled in the art can easily understand that similar effects are obtainable using a ghrelin homolog having threonine as the position-3 amino acid, by applying the present invention.

### (1) Regulator for physiological functions of ghrelin

According to the definition above, examples of "regulator for physiological functions of ghrelin" include a substance which has a fatty acid moiety capable of forming an ester with the hydroxyl group of position-3 amino acid (for example, Ser (3)) of ghrelin molecule and regulating at least one function of ghrelin.

The fatty acid that can be used as an active ingredient of the regulator of the present invention includes saturated or unsaturated fatty acids of carbon number 2 to 35. Specific examples include those having even carbon number such as butanoic acid (C4), hexanoic acid (C6), octanoic acid (C8), decanoic acid (C10), dodecanoic acid (C12), tetradecanoic acid (C14), hexadecanoic acid (C16) and octadecanoic acid (C18); those having odd carbon number such as pentanoic acid (C5), heptanoic acid (C7), nonanoic acid (C9), pentadecanoic acid (C15) and heptadecanoic acid (C17); and monoenoic or polyenoic fatty acids thereof. Fatty acids of carbon number 4 to 18 are preferred, those of carbon number 6 to 16 are more preferred, and those of carbon number 6 to 12 are most preferred, but not limited thereto. In addition, the regulators of the present invention can be used in one kind alone or in a mixture of plural kinds of the above-mentioned fatty acids.

In the case where the regulators are "positive regulators" to increase (elevate) the physiological functions of ghrelin, fatty acids of carbon number 4 to 12, preferably 6 to 12, more preferably 8 to 10 are generally usable, although it varies depending on the subjective animal.

The "positive regulator" having the activity of increasing the "activated ghrelin" concentration, it has almost the same meaning as the "accelerator for the formation of activated ghrelin".

In the case where the regulator is "negative regulator" to suppress the physiological functions of ghrelin, fatty acids other than those exemplified as positive regulators above are generally usable, but not limited thereto.

It is evident that the above-mentioned examples are not definitive and the preferable range varies depending on the subjective animal, and modified ghrelin having or lacking physiological activity of ghrelin can be produced using a fatty acid of either longer or shorter carbon chain than that mentioned above. Such a fatty acid or a derivative thereof is also encompassed within the scope of the present invention.

Regarding the activity of promoting growth hormone secretion that is one of physiological functions of ghrelin, it has been known that, when the subject is human, ghrelin modified at the position-3 amino acid residue with octanoic acid (carbon number (C)8) and/or ghrelin modified with decanoic acid (carbon number (C)10) has activity of promoting growth hormone secretion, namely acts as a positive regulator (an activated ghrelin), whereas ghrelin modified at the position-3 amino acid residue with hexanoic acid (carbon number (C)6) does not affect the growth hormone secretion, namely acts as a negative regulator.

In this case, a fatty acid of 8 to 10 carbon number can be preferably used as an accelerator for the formation of activated ghrelin of the present invention. In particular, octanoic acid of carbon number 8 has a property to form an ester with the hydroxy group of the position-3 amino acid more easily as compared to decanoic acid of carbon number 10, and therefore can be used as an effective accelerator for the formation of activated ghrelin.

In the above, a preferred accelerator for the formation of activated ghrelin for human as the subject is described in regard to the activity of promoting growth hormone secretion, whereas it is evident that the activated ghrelin differs depending on the subjective animal and the physiological action of ghrelin, and, therefore, a fatty acid having a longer or shorter carbon chain than that mentioned above can be used as an accelerator for the formation of activated ghrelin. Such a fatty acid or a derivative thereof is encompassed within the scope of the present invention.

Examples of a "derivative of fatty acid" that is an active ingredient of regulators include derivatives of the above-mentioned fatty acids in any form, which can liberate the above-mentioned fatty acids, or can form an ester with the hydroxyl group of position-3 amino acid of ghrelin molecule in the living body. Such a derivative also may be converted as appropriate into a form of salt or ester for the purpose of improving the solubility, the absorbability from gastrointestinal tract, the taste, and odor. The method for production of such a derivative is well known in the production field in relation to medicines, foods, feeds and the like, and a person skilled in the art can easily produce a suitable derivative in accordance with the object.

Preferred examples of "derivative of fatty acid" include esters with mono- or poly-alcohols which are normally used for a similar purpose. In particular, glycerin is a preferred alcohol. In the case of glycosides, mono-, di- or tri-glycerides, or a mixture thereof may be used, and triglyceride approved as a food is preferred, but not limited thereto.

A fatty acid or a derivative thereof as an active ingredient of the regulator of the present invention can be produced by a method known to a person skilled in the field of organic chemistry, or is available from commercial suppliers.

### (2) Physiological Functions of Ghrelin

Examples of physiological functions of ghrelin that can be regulated by the regulator of the present invention include all physiological functions of acyl ghrelin, for example, activity of increasing an intracellular calcium ion concentration, activity of promoting growth hormone secretion, activity of promoting eating, regulatory activity relating to fat accumulation, activity of ameliorating cardiac function or activity of stimulating gastric acid secretion. In particular, it deeply participates in, but not limited thereto, release of growth hormone, stimulation of appetite, induction of adiposity, amelioration of cardiac functions, gastric acid secretion and the like. The positive regulator of the present invention improves the physiological functions of ghrelin, and hence exhibits similar effects to ghrelin or its analog. In other words, the positive regulator can exhibit various effects such as promotion of growth hormone secretion, stimulation of eating, induction of obesity, amelioration of cardiac function, stimulation of gastric acid secretion, and the like.

### (3) Use of Regulators for Physiological Functions of Ghrelin, 1 (Pharmaceutical Composition)

The regulator of the present invention can be used as a medicine expressing the above-mentioned effects for mammals, birds, fishes, amphibians and the like, for example, human, swine, cattle, horse, sheep, rabbit, rat, mouse, dog, chicken, eel, rainbow trout, frog and the like.

Specifically, the regulator of the present invention is useful as a therapeutic agent for eating disorder, an agent for promoting growth hormone secretion, a therapeutic agent for cardiac disease, a therapeutic agent for stomach functional disease, a protecting agent for intestinal mucosa or an agent for prevention of small intestinal mucosa disorder at the time of intravenous nutrition, a therapeutic agent for osteoporosis, an agent for decreasing cachexy involved in chronic diseases, a therapeutic agent for pulmonary insufficiency, and the like. In particular, the regulator of the present invention is useful for prevention or treatment of osteoporosis, anorexia, cardiac disease, rheumatism and inflammatory intestine disease, and also for promoting postoperative recovery in human.

The fatty acid or its derivative of the present invention *per se* functions as a regulator for physiological functions of ghrelin of the present invention, and therefore can be used as it is. However, it is preferably formulated in an appropriate form (including a liquid or solid form by a method known in the art) for the sake of convenience in handling or application. Examples include a liquid preparation and a suspension in an aqueous or non-aqueous medium (diluent), as well as powder, granules or tablets comprising a physiologically acceptable or pharmaceutically acceptable carrier. Such a pharmaceutical composition can promote or suppress the function of ghrelin in a variety of animal species described, for example, in the section of "Physiological Functions of Ghrelin" above, and can exhibit the therapeutic effects described in the same section.

In the case where the regulator for physiological functions of ghrelin of the present invention is formulated in a pharmaceutical composition, the preparation can be carried out by a method *per se* known to a person skilled in the art using an excipient, solvent, carrier, a preservative, and the like.

The pharmaceutical composition of the present invention can be administered through an oral or parenteral route (for example, an intracutaneous, subcutaneous or intravenous injection, an infusion or the like), by a method known in the field of medicine or animal medicine.

The dose of the regulator of the present invention varies depending on various factors including the selected fatty acid or its derivative, the administration route, and the conditions of the subject to be treated such as the disorder to be treated, the age, the body weight, and the like, and is normally determined by a doctor. On the basis of the fatty acid, the dose may be from 0.0001 mg to 1000 mg, preferably 0.001 mg to 100 mg, and more preferably 0.01 mg to 10 mg, but not limited to such a range. Also, in the case where the subject is an animal other than human, the dosage is determined as appropriate by a veterinarian or the like depending on the subject.

### (4) Use of Regulators for Physiological Functions of Ghrelin, 2 (Functional Foods)

The regulators of the present invention, since they have no worry about side effects, can be routinely used as functional foods for promotion or suppression of appetite, dissolution of obesity, improvement of malnutrition and the like. In particular, the regulators of the present invention can be used for the control of health conditions of mammals through the control of the body weight or the like, and further for the growth acceleration of animals, the reduction of fatty meat in the meat and the like. Thus, the regulators of the present invention are useful also in the stock farming, the poultry farming, the culture fishery and the like.

In the present specification and claims, the term "functional food" is used in a broad sense, and refers to food which animals including human can take with expectation of some physiological functions, except those defined as medicines. Specific examples include foods in the form of, for example, supplements which contain a fatty acid or its derivative of the present invention that is a regulator for physiological functions of ghrelin of the present invention as an active ingredient, and also foods prepared by compounding a fatty acid or its derivative of the present invention that is a regulator for physiological functions of ghrelin of the present invention as one ingredient into a general food so as to provide it with activity of regulating physiological functions of ghrelin in the living body. Such functional foods can be exemplified by health promoting foods, conditioned specific health promoting foods, food supplements, dietary supplements, and also as foods accompanied by an indication saying that they are to be used for regulating the physiological functions of ghrelin (to promote the formation of activated ghrelin) in the living body, or for preventing or suppressing disorders associated with the physiological functions.

The fatty acid or its derivative of the present invention *per se* functions as a regulator for physiological functions of ghrelin of the present invention, and therefore can be used as a functional food as it is. However, it is preferably formulated in an appropriate form (including a liquid or solid form by a method known in the art) for the sake of convenience in handling or application. Examples include a liquid preparation and a suspension in an aqueous or non-aqueous medium (diluent), as well as powder, granules or tablets comprising a physiologically acceptable or pharmaceutically acceptable carrier.

There are no limitations regarding the objects of the functional foods containing the above-mentioned fatty acid or its derivative of the present invention as a ingredient. Specific examples include frozen deserts such as ice cream, ice milk, lacto ice, sherbet, ice and the like; beverages such as a milk beverage, a lactic acid bacteria beverage, a soft drink (including one containing fruit juice), a carbonated drink, a fruit juice drink, a vegetable drink, a vegetable/fruit drink, a sport drink, a powder drink and the like; alcoholic beverages such as a liqueur and the like; tea beverages such as a coffee drink, a black tea drink and the like; soups such as a consommé soup, a pottage soup and the like; puddings such as a custard pudding, a milk pudding, a pudding containing fruit juice, and the like; deserts such as jelly, Bavarian cream, yoghurt and the like; gums (bar gums and sugar-coated granular gums) such as a chewing gum, a bubble gum and the like; chocolates such as flavored chocolates like a strawberry chocolate, a blueberry chocolate and a melon chocolate, besides coated chocolates like a marble chocolate, and the like; caramels such as hard candies (including bonbon, butter ball, marble and the like), soft candies (including caramel, nougat, wolf willow candy, marshmallow and the like), a drop, a taffy and the like; bake goods such as hard biscuits, cookies, Okaki (baked rice cakes), Senbei (rice crackers) and the like; sauces such as a separate dressing, a non-oil dressing, a ketchup, a baste, a sauce and the like; jams such as strawberry jam, blueberry jam, marmalade, apple jam, apricot jam, preserves and the like; fruit wines such as red wine and the like; processed meat products such as a ham, a sausage, roast pork and the like; marine paste products such as a fish ham, a fish sausage, fish paste, Kamaboko (heated fish paste), Chikuwa (baked short pipe-shaped fish paste), Hanpen (pounded fish cake), Satsumaage (fried fish paste ball), Datemaki (roasted and rolled fish paste), whale bacon and the like; dairy products such as cheese and the like; noodles such as Japanese wheat noodle, Hiyamugi (cold Japanese noodle), Soumen (fine noodle), buckwheat noodle, Chinese noodle, spaghetti, macaroni, rice noodle, Harusame (bean-starch vermicelli), won ton and the like; as well as a variety of other processed foods such as various daily dishes, Fu (wheat-gluten bread), Denbu (mashed and seasoned fish) and the like.

The content of the regulator of the present invention in the functional foods can be determined as appropriate by a person skilled in the art in consideration of factors such as the kind and shape of the product, the subject taking the same (species and age bracket) and the like by referring to the dosage described in the above-mentioned section regarding the pharmaceutical composition.

The "positive regulator" of the present invention, specifically the "accelerator for the formation of activated ghrelin", is extremely useful as a functional food having activity of increasing an intracellular calcium ion concentration and activity of promoting growth hormone secretion. Such a functional food can efficiently exert physiological effects through the activity of promoting growth hormone secretion, for example, strengthening of muscles/skeletons, decrease of fats, rejuvenescence of skin, refreshment and the like, without raising worry of side effects.

As stated above, the accelerator for the formation of activated ghrelin is comprised of a medium-chain fatty acid of 6 to 12 carbon number or its derivative. In particular, when the subject is human, the agent comprises a medium-chain fatty acid of preferably 8 to 10 carbon number, most preferably 8 carbon number, or a derivative thereof.

### (5) Use of Regulators for Physiological Functions of Ghrelin, 3 (Method for Prevention or Treatment)

The method for preventing or treating disorders associated with the physiological functions of ghrelin by the use of the regulator of the present invention can be performed according to a method known in the art by administering the regulator itself, or a pharmaceutical composition or a functional food containing the same to a human or a nonhuman animal.

### EXAMPLES

The present invention is further illustrated by the following examples, but is not limited by these examples in any respect.

### Abbreviations

GH: Growth hormone
GHS: Growth hormone secretagogue
GHS-R: Growth hormone secretagogue receptor
Ir: Immunoreactivity
RIA: Radioimmunoassay
CHO: Chinese hamster ovary
[Ca²⁺]i: Intercellular calcium concentration
AcOH: Acetic acid
HPLC: High-performance liquid chromatography
RP: Reverse-phase
MALDI-TOF-MS: Matrix-assisted laser desorption/ionization time-of-flight mass spectrometry
N-RIA: Radioimmunoassay of the N-terminal fragment of *n-*octanoyl ghrelin [D1-D11]
C-RIA: Radioimmunoassay of the C-terminal fragment of ghrelin [D 13-D28]
MCFA: Medium-chain fatty acid
MCT: Medium-chain triglyceride
LCFA: Long-chain fatty acid
LCT: Long-chain triglyceride
SCT: Short-chain triglyceride

### Example 1

### (1) Materials and Methods

### 1) Radioimmunoassay of ghrelin

RIAs specific for ghrelin were performed as previously described [D2]. Two polyclonal antibodies were raised in rabbits against the N-terminal (Gly¹-Lys¹¹ with *O*-*n*-octanoylation at Ser³) and C-terminal (Gln¹³-Arg²⁸) fragments of rat ghrelin. RIA incubation mixtures were composed of 100 µl of either standard ghrelin or an unknown sample, with 200 µl of antiserum diluted in RIA buffer (50 mM sodium phosphate buffer (pH 7.4), 0.5% BSA, 0.5% Triton-X100, 80 mM sodium chloride (NaCl), 25 mM disodium ethylenediamine-tetraacetate (EDTA 2-Na) and 0.05% sodium azide (NaN₃)) containing 0.5% normal rabbit serum. Anti-rat ghrelin [D1-D11] and anti-rat ghrelin [D13-D28] antisera were used at final dilutions of 1/3,000,000 and 1/20,000, respectively. After a 12-hour incubation at 4°C, 100 µl of ¹²⁵I-labeled ligand (20,000 cpm) was added for an additional 36-hour incubation. Then, 100 µl of anti-rabbit goat antibody was added. After incubation for 24 hours at 4°C, free and bound tracers were separated by centrifugation at 3,000 rpm for 30 min. Pellet radioactivity was quantified in a gamma counter (ARC-600, Aloka, Tokyo). All assays were performed in duplicate at 4°C.

Both types of the antisera exhibited complete cross-reactivity with human, mouse and rat ghrelins. The anti-rat ghrelin [D1-D11] antiserum, which specifically recognizes the Ser³ *n*-octanoylated portion of ghrelin, did not recognize a des-acyl ghrelin. The cross-reactivity of N- RIA to *n*-decanoyl ghrelin and that to *n*-hexanoyl ghrelin are 20% and 0.3%, respectively. Anti-rat ghrelin [D13-D28] antiserum equally recognized both des-acyl and all acylated forms of ghrelin peptide. In the following sections, the RIA system using the antiserum against the N-terminal fragment of rat ghrelin [D1-D11] is termed N-RIA, while the RIA system using antiserum against the C-terminal fragment [D13-D28] is termed C-RIA.

### 2) Calcium mobilization assay of ghrelin

Before the assay, CHO-GHSR 62 cells [D1] stably expressing rat GHS-R (ghrelin receptor) were plated for 12-15 hours in flat-bottom, black-walled 96-well plates (Coming Costar Corporation, Cambridge, MA) at 4 x 10⁴ cells/well. Cells were then preincubated for 1 hour with 4 µM Flo-4-AM-fluorescent indicator dye (Molecular Probes, Inc., Eugene, OR) dissolved in assay buffer (Hanks' balanced salts solution (HBSS), 10 mM HEPES, 2.5 mM probenecid) supplemented with 1% fetal calf serum (FCS). After washing four times with assay buffer, samples, each dissolved in 100 µl basic buffer with 0.01% bovine serum albumin, were added to the prepared cells. Changes in the intracellular calcium concentration were measured using a FLEX station (Molecular Devices, Sunnyvale, CA).

### 3) Preparation of stomach samples for ghrelin assay

Stomachs collected from either mice or rats were washed two times in phosphate buffered physiological saline (pH 7.4). After measuring the wet weight of each sample, the whole stomach tissue was diced and boiled for 5 minutes in a 10-fold volume of water to inactivate intrinsic proteases. After cooling on ice, boiled samples were adjusted to 1 M acetic acid-20 mM hydrochloric acid (HC1). Peptides were extracted following homogenization with a Polytron mixer (PT 6100, Kinematica AG, Littan-Luzern, Switzerland),. Extract supernatants, isolated following 15-minute-centrifugation at 15,000 rpm (12,000 x g), were lyophilized and stored at -80°C. The lyophilized samples were redissolved in either RIA buffer or calcium mobilization assay buffer prior to ghrelin RIA or calcium mobilization assay, respectively.

### 4) Preparation of plasma samples for ghrelin assay

Plasma samples were prepared as previously described [D2]. Whole blood samples were immediately transferred to chilled polypropylene tubes containing EDTA-2 Na (1 mg/ml) and aprotinin (1,000 kallikrein inactivator units/ml) and centrifuged at 4°C. Immediately after separation of the plasma, hydrogen chloride was added to the sample at final concentration of 0.1 N and then diluted with an equal volume of physiological saline. The sample was loaded onto a Sep-Pak C18 cartridge (Waters, Milford, MA) pre-equilibrated with 0.1% trifluoroacetic acid (TFA) and 0.9% NaCl. The cartridge was washed with 0.9% NaCl and 5% acetonitrile (CH₃CN)/0.1% TFA, and then eluted with 60% CH₃CN/0.1% TFA. The eluate was then lyophilized and residual materials were redissolved in 1 M acetic acid (AcOH) and adsorbed onto a SP-Sephadex C-25 column (H⁺-form, Pharmacia, Uppsala, Sweden) pre-equilibrated in 1 M AcOH. Successive elution with 1 M AcOH, 2 M pyridine, and 2 M pyridine-AcOH (pH 5.0) provided three fractions: SP-I, SP-II and SP-III. The SP-III fraction was evaporated and redissolved in 1M AcOH, separated by C18 RP-HPLC (Symmetry 300, 3.9 x 150 mm, Waters) with a linear gradient from 10 to 60% CHsCN/0.1% TFA at a flow rate of 1.0 ml/minute for 40 minutes. Five hundred micro-litter fractions were collected. Ghrelin peptide content in each fraction was measured by ghrelin C-RIA as described above.

### 5) Concentration and acyl modification of ghrelin after free fatty acid or triacylgycerol (medium-chain triglyceride) ingestion

Male C57BL/6J mice weighing 20-25 g (10-12 week old) were maintained under controlled temperature (21-23°C) and light conditions (light on 0700-1900) with *ad libitum* access to food and water. Medium-chain fatty acids (MCFAs), i.e., *n*-hexanoic, *n*-octanoic, and *n-*lauric acids (Sigma-Aldrich Japan Co., Ltd., Tokyo), were dissolved in water at 5 mg/ml. *n*-Palmitic acid, a common long-chain fatty acid (LCFA) (Sigma-Aldrich Japan Co., Ltd., Tokyo), was mixed into standard laboratory chow (CLEA Rodent Diet CE-2, CLEA Japan, Osaka) at a concentration of 1% (w/w), to equilibrate the total intake amount of this lipid to the other medium-chain fatty acids contained in the food. Medium- and long-chain triglycerides (MCTs and LCTs), i.e., glyceryl trihexanoate, trioctanoate, tridecanoate and tripalmitates (Wako Pure Chemical, Osaka, Japan), were mixed with standard laboratory chow at a concentration of 5% (w/w). Whole stomach tissues from treated mice were collected at the indicated times (0-14 days) after ingesting the free fatty acid- or triacylglyceride-containing food. Fresh tissue samples from these mice were diced and boiled for 5 minutes in a 10-fold volume of water. The tissue-containing solution was then adjusted to 1M acetic acid after cooling and then homogenized with a Polytron mixer. The supernatant, obtained after centrifugation at 15,000 rpm for 15 minutes, was then lyophilized. The lyophilized material was dissolved in RIA buffer and subjected to ghrelin C- and N-RIA. To elucidate the forms of ghrelin peptides modified by different acyl groups, extracted stomach peptides were collected using a Sep-Pak Plus C18 cartridge (Waters, Milford, MA) and subjected to C18 RP-HPLC (Symmetry 300, 3.9 x 150 mm, Waters) with a linear gradient from 10 to 60% CH₃CN/0.1% TFA at a flow rate of 1.0 ml/minute for 40 minutes. Five hundred micro-litter fractions were collected. Ghrelin peptide content in each fraction was measured by ghrelin C- and N-RIA as described above. Degradation of ghrelin was not observed during the extraction.

### 6) Northern blot analysis

Total RNAs were extracted from the stomachs of male C57BL/6J mice (12 week old) by acid guanidium thiocyanate-phenol chloroform extraction [D24] using TRIzol reagent (Invitrogen, Carlsbad CA, USA). Two µg of total RNA was electrophoresed through a 1% agarose gel containing formaldehyde, and then transferred to a Zeta-Probe blotting membrane (Bio-Rad Laboratories, Hercules, CA). Membranes were hybridized with a ³²P-labeled rat ghrelin cDNA probe in hybridization buffer containing 50% formamide, 5 x SSPE, 5 x Denhardt's solution, 1% sodium dodecyl sulfate (SDS), and 100 µg/ml denatured salmon sperm. After an overnight hybridization at 37°C, membranes were washed and exposed to BioMax-MS film (Eastman Kodak, Rochester, NY) for 12 hours at -80°C. Ghrelin mRNA levels were quantified using a Bioimaging analyzer BAS 2000 (Fujix, Tokyo, Japan).

### 7) Purification of n-heptanoyl ghrelin

*n*-Heptanoyl ghrelin was purified using the same method as described for previous ghrelin purification by anti-rat ghrelin [D1-D11] IgG immunoaffinity chromatography [D22]. During purification, ghrelin activity was assayed by measuring changes in intracellular calcium concentration within a cell line stably expressing rat GHS-R (ghrelin receptor) (CHO-GHSR62) using a FLEX station (Molecular Devices, Sunnyvale, CA). Ghrelin C-RIA system was also used to monitor ghrelin immunoreactivity in samples.

Male C57BL/6J mice weighing 20-25 g (10-12 week old) were maintained under controlled temperature (21-23°C) and light conditions (light on 0700-1900) with *ad libitum* access to food and water. Glyceryl triheptanoate (Fluka Chemie GmbH, Buchs, Switzerland) was mixed with standard laboratory chow at a concentration of 5% (w/w). Four days after mice were fed glyceryl triheptanoate-containing food, stomachs (total 1,000 mg) were recovered from mice (n = 7). The total consumption of glyceryl triheptanoate-containing food was approximately 13.5 g/mouse, amounting to 675 mg total glyceryl triheptanoate ingested by each mouse. Stomachs were minced and boiled for 5 minutes in 5 x volumes of water to inactivate intrinsic proteases. The stomach tissue solution was then adjusted to 1M acetic acid (AcOH)-20 mM HCl and homogenized in a Polytron mixer. Supernatants of these extracts, obtained after a 30-minute-centrifugation at 20,000 rpm, were loaded onto a cartridge of Sep-Pak C18 environmental cartridge (Waters, Milford, MA) pre-equilibrated in 0.1% trifluoroacetic acid (TFA). After washing with 10% acetonitrile (CH₃CN)/0.1% TFA, the peptide fraction was eluted in 60% CH₃CN/0.1% TFA. The eluate was evaporated and lyophilized. Residual materials were redissolved in 1 M AcOH and adsorbed onto a SP-Sephadex C-25 column (H⁺-form, Pharmacia, Uppsala, Sweden) pre-equilibrated in 1 M AcOH. Successive elution with 1 M AcOH, 2 M pyridine, and 2 M pyridine-AcOH (pH 5.0) provided three fractions: SP-I, SP-II and SP-III. After applying the lyophilized SP-III fraction to a Sephadex G-50 fine gel-filtration column (1.9 x 145 cm) (Pharmacia, Uppsala, Sweden), 5 ml fractions were collected. A portion of each fraction was subjected to the ghrelin calcium-mobilization assay using CHO-GHSR 62 cells. Half of the isolated active fractions (# 47-51), collected using a Sep-Pak C18 light cartridge, was lyophilized, dissolved in 1.0 ml of 100 mM phosphate buffer (pH 7.4), and subjected to anti-rat ghrelin [D1-D11] IgG immunoaffinity chromatography. Adsorbed substances were eluted in 500 µl of 10% CH₃CN/0.1% TFA. The eluate was evaporated, then separated by RP-HPLC (Symmetry 300, 3.9 x 150 mm, Waters, Milford, MA). *n*-Heptanoyl-modified ghrelin was purified at a retention time of 18.4 minutes and subjected to a mass spectrometry to determine the molecular weight. The amino acid sequences of purified peptides were analyzed with a protein sequencer (494, Applied Biosystems, Foster City, CA).

### 8) Mass spectrometric analysis of n-heptanoyl ghrelin

Matrix-assisted laser desorption/ionization time-of-flight mass spectrometry (MALDI-TOF-MS) was performed using a Voyager DE-Pro spectrometer (Applied Biosystems, Foster City, CA) [D25]. Mass spectra were recorded in the reflector mode, with an accelerating voltage of 20 kV. Saturated α-cyano-4-hydroxycinnamic acid in 60% acetonitrile (CH₃CN) and 0.1% trifluoroacetic acid (TFA) was used as working matrix solution. Approximately 1 pmol of the final purified sample was mixed with matrix solution, placed on the sample probe, and dried in air prior to analysis. All mass spectra were acquired in positive ion mode, averaged by 100 spectra.

### (2) Experiments and Results

### 1) Effect of free fatty acid ingestion for the stomach content of n-octanoyl ghrelin

To examine the effect of free fatty acid ingestion on the acyl-modification of ghrelin, gastric peptides were extracted from mice fed *n*-hexanoic acid (C6), *n*-octanoic acid (C8), *n*-lauric acid (C12), or *n-*palmitic acid (C16) and water, and normal control mice (Control) fed with standard chow and water. After ingestion, the concentrations of acyl-modified and total (acyl-modified plus des-acyl) ghrelins were measured. While acyl-modified ghrelin was measured by N-RIA, total ghrelin was measured by C-RIA. The results are shown in Fig. 1.

A represents acyl-modified ghrelin concentrations measured by ghrelin N-RIA (n=8). As N-RIA is very specific for acyl-modified ghrelin and the main form of acylated ghrelin is *n*-octanoyl ghrelin, the acyl-modified ghrelin concentration measured by N-RIA primarily reflects *n*-octanoyl ghrelin population. B represents total ghrelin concentration measured by ghrelin C-RIA (n=8). The total ghrelin concentration includes both acylated and des-acyl ghrelins. C represents the ratio of acyl-modified/total ghrelin. Data represent mean ± S.D. of ghrelin concentrations in stomach extracts (from 1 mg wet weight). Asterisks indicate statistical significance. *, p<0.01; **, p<0.001 vs. control.

Figure 1 shows that the stomach content of *n*-decanoyl and *n-*hexanoyl ghrelins was low in comparison to *n*-octanoyl ghrelin and the cross-reactivity of N-RNA for *n*-decanoyl- and *n*-hexanoyl-modified ghrelins are 20% and 0.3%, respectively. This means that the concentration of acyl-modified ghrelin measured by N-RIA primarily reflects the *n*-octanoyl ghrelin. During the experimental period (0-14 days), no significant differences in either mouse body weight or total dietary consumption were observed between the fatty acid-ingesting and control groups.

After mice were given *n*-hexanoic acid, *n*-octanoic acid, *n*-lauric acid, or *n*-palmitic acid for 14 days, the gastric concentrations of acyl-modified and total ghrelins were compared with the concentrations in control mice fed normal chow and water. The gastric concentration of acyl-modified ghrelin increased significantly in mice fed *n*-octanoic acid (Fig. 1A). The mean concentration of acyl-modified ghrelin in the stomach was 1,795 fmol/mg wet weight in control rats fed normal food (n=8) and 2,455 fmol/mg wet weight in mice fed *n*-octanoic acid-containing food (n=8), respectively. No significant changes were observed in the total ghrelin concentration measured by C-RIA (Fig. 1B). Therefore, the ratio of *n*-octanoyl ghrelin/total ghrelin increased in mice fed *n*-octanoic acid (Fig. 1C). No significant changes were detected in the contents of either acyl-modified or total ghrelins in the stomachs of mice fed *n*-hexanoyl, *n*-decanoyl, or *n*-palmitic acids. Thus, the exogenously supplied *n*-octanoic acid increased gastric concentrations of *n*-octanoyl ghrelin without increasing the total (acyl-modified and des-acyl) ghrelin peptide. These results suggest that the ingested *n-*octanoic acid stimulated acyl modification of ghrelin.

### 2) Effect of medium- to long-chain triacylglycerol (triglyceride) ingestion on the stomach content of acyl-modified ghrelin

Orally ingested triacylglycerols are intraluminally hydrolyzed and absorbed through the gastro-intestinal mucosa as free fatty acids or monoglycerides. Thus, ingested triacylglycerols may serve as a source of free fatty acids [D26]. To examine if ingested triacylglycerols are used for acyl-modification of ghrelin, mice were fed chow mixed with 5% (w/w) glyceryl trihexanoate (C6), trioctanoate (C8), tridecanoate (C10), and tripalmintate (C16). After two weeks, gastric peptides were extracted. Ghrelin concentration in the stomach of these mice and in that from mice fed standard laboratory chow (Control) (n=8) are shown. The content of acyl-modified and total ghrelins was measured by N- and C-RIA. The results are shown in Fig. 2.

A represents acyl-modified ghrelin concentrations measured by ghrelin N-RIA. B represents total ghrelin concentration measured by ghrelin C-RIA. Data represent mean ± S.D. of ghrelin concentration in stomach extracts (from 1mg wet weight) (n=5). C represents the ratio of acyl-modified/total ghrelin concentration. Data represent mean ± S.D. of calculated ratios (n=5). Asterisks indicate statistical significance. *, p<0.05; **, p<0.01 vs. control.

Figure 2 shows that glyceryl trioctanoate ingestion stimulated production of acyl-modified ghrelin in stomach tissue (Fig. 2A). In contrast, glyceryl trihexanoate ingestion slightly suppressed acyl-modified ghrelin production. However, mice fed glyceryl trihexanoate exhibited increased concentrations of *n*-hexanoyl ghrelin (Fig. 2A), (Table 1). Ingestion of glyceryl tridecanoate and glyceryl tripalmilate had no effect on the production of acyl-modified ghrelin (Fig. 2A). Furthermore, no significant changes in the total stomach concentration of ghrelin (des-acyl and acyl-modified ghrelins) could be detected within five independent groups of mice (Fig. 2B). Thus, the molar ratio of acyl-modified ghrelin/total ghrelin decreased significantly in glyceryl trihexanoate-treated mice and increased in glyceryl tridecanoate-treated mice (Fig. 2C). During the experimental period (0-2 weeks), no significant differences in body weight or total food consumption could be observed between triacylglycerol-fed and control groups.

### 3) Molecular forms of ghrelin peptide after triglycerol ingestion

To clarify what molecular forms of ghrelin peptide are present after the ingestion of triacylglycerol (triacylglycerin), peptide extracts from stomachs of mouse fed chow containing glyceryl trihexanoate (C6:0-MCT), glyceryl trioctanoate (C8:0-MCT), glyceryl tridecanoate (C10:0-MCT), and standard laboratory chow (Control) were fractionated by HPLC, and measured for ghrelin immunoreactivity by C-RIA. This analysis revealed the molecular forms of ghrelin in stomach extracts from mice fed glyceryl trihexanoate, trioctanoate, and tridecanoate (Fig. 3). Based on the observed retention times of synthetic acyl-modified ghrelin peptides, peaks a, d, h, and k corresponded to des-acyl ghrelin, peaks b, f, i, and 1 corresponded to n-octanoyl (C8:0) ghrelin, and peaks c, g, j, and m corresponded to n-decenoyl (C10:1) ghrelin.

Ingestion of glyceryl trioctanoate stimulated the production of *n-*octanoyl ghrelin (peak i in Fig. 3). The molar ratio of *n*-octanoyl/total ghrelin was over 60% in treated mice (Table 1). This high *n*-octanoyl ghrelin ratio was not observed in mice fed normal food and water (Table 1). The stomach content of *n*-octanoyl ghrelin also increased after *n-*octanoic acid ingestion, indicating that both glyceryl trioctanoate and *n-*octanoic acid stimulated the production of *n*-octanoyl ghrelin.

*n*-Hexanoyl ghrelin could only be detected at very low levels in stomach of mice fed normal chow. When mice were fed glyceryl trihexanoate, however, the stomach concentration of *n*-hexanoyl ghrelin increased drastically (peak e). In these mice, significant decreases in *n-*octanoyl ghrelin concentration were also detected (peak f in Fig. 3 and Table 1) in comparison to the levels observed in control mice (peak b in Fig. 3 and Table 1). The content of *n*-hexanoyl ghrelin also increased after *n*-hexanoic acid ingestion (data not shown).

Moreover, when mice were fed glyceryl tridecanoate, the stomach concentration of *n*-decanoyl ghrelin was increased (peak n).

In addition, ghrelin peaks that eluted at the same retention times as synthetic *n*-butanoyl (C4:0), *n*-dodecanoyl (C12:0), and *n-*palmitoyl (C 16:0) ghrelin were not observed in stomach extracts of mice given glyceryl tributyrate, trilaurate, or tripalmitate (data not shown). These data indicate that neither glyceryl tributyrate nor tripalmitate were transferred to ghrelin in mice.

**Table 1: Concentrations of des-acyl and acyl-modified ghrelin peptides in the stomachs of mice after ingestion of medium-chain (C6:0-C10:0) triglycerides**

| | Des-acyl ghrelin | C6:0-ghrelin | C8:0-ghrelin | C10:1-ghrelin * | C10:0-ghrelin |
|---|---|---|---|---|---|
| Control | 285.6±20.4 | 25.9±1.4 | 514.9±28.7 | 180.7±17.7 | 20.0±2.8 |
| C6:0-MCT | 245.0±11.0 | 237.3±35.0^{a)} | 358.0±33.0^{b)} | 133.5±12.1^{b)} | 14.1±5.2 |
| C8:0-MCT | 236.6±21.1 | 12.4±4.9 | 774.0±89.1^{c)} | 51.5±13.2^{a)} | 8.1±3.1 |
| C10:0-MCT | 181.4±31.5^{c)} | 22.1±6.1 | 460.0±70.6 | 64.5±14.2^{b)} | 95.1±9.7^{a)} |

Male C57BL/6J mice were fed chow mixed with 5% (w/w) glyceryl trihexanoate (C6:0-MCT), glyceryl trioctanoate (C8:0-MCT), or glyceryl tri-decanoate (C10:0-MCT) for 14 days. The concentrations of des-acyl ghrelin, *n*-hexanoyl ghrelin (C6:0-ghrelin), *n*-octanoyl ghrelin (C8:0-ghrelin), *n*-lecenoyl ghrelin (C10:1-ghrelin), and *n-*decanoyl ghrelin (C 10:0-ghrelin) from stomach samples (from 0.2 mg wet weight) were measured by ghrelin C-RIA after HPLC fractionation. Data represent mean ± S.D. of quadruplicate samples. a) : p<0.001, b) : p<0.05 and c) : p<0.01 vs. control.
(*: After purification, at least two unidentified ghrelin molecules independent of *n*-decenoyl ghrelin were observed in the same fraction.)

### 4) Time course of acyl-modified ghrelin production after glyceryl trioctanoate ingestion

To examine time-dependent changes in the production of *n-*octanoyl ghrelin after the ingestion of a glyceryl trioctanoate, mice were fed with glyceryl trioctanoate-containing chow (5%, w/w) after a 12-hour fasting period. The concentrations of acyl-modified and total ghrelins in the stomach were measured at the indicated times. The results are shown in Fig. 4.

A represents acyl-modified ghrelin content measured by ghrelin N-RIA. B represents total ghrelin content measured by ghrelin C-RIA. After 12-hour fasting, glyceryl trioctanoate (5% w/w)-containing chow was given to mice beginning at the time (0 hour) indicated by the arrow. Stomach samples were isolated from control mice fed standard laboratory chow (closed circles) and mice fed glyceryl trioctanoate (open circles) at the indicated times. Each point represents mean ± S.D. (n = 8). Asterisks indicate statistical significance. *, p<0.05; **, p<0.01 and ***, p<0.001 vs. control.

Figure 4 clearly shows that the content of acyl-modified ghrelin in the stomach increased three hours after the ingestion of glyceryl trioctanoate. When continuously supplied with glyceryl trioctanoate, the concentrations of *n*-octanoyl ghrelin in the stomach of mouse increases. The concentrations gradually increased to maximal levels at 24 hour after starting ingestion. The stomach concentration of acyl-modified ghrelin 14 days after ingestion remained significantly higher than that of mice fed normal chow (Fig. 4A). In contrast, no significant changes in the stomach content of total ghrelin, measured by C-RIA, were observed under these conditions (Fig. 4B).

### 5) Ghrelin mRNA expression after glyceryl trioctanoate ingestion

To examine if the ingestion of MCTs affects the expression of ghrelin mRNA, mouse stomach RNA was quantitated by Northern blot analysis after 4 days of glyceryl trioctanoate ingestion. The results are shown in Fig. 5.

Each lane contained 2 µg of total RNA. The lower panel indicates 28S and 18S ribosomal RNAs internal controls.

Figure 5 shows that the expression level of gastric ghrelin mRNA did not change after the ingestion of glyceryl trioctanoate. Furthermore, as the ingestion of glyceryl trioctanoate increased the content of *n*-octanoyl ghrelin in the stomach without changing the total ghrelin content, ingestion of glyceryl trioctanoate stimulated only the octanoyl modification step of ghrelin peptide synthesis.

### 6) Molecular forms of ghrelin peptides after glyceryl triheptanoate ingestion

To examine the possibility that ingested free fatty acids are used for the direct acyl modification of ghrelin, mice were fed medium-chain triglycerides (MCTs) that are neither present in natural food sources nor synthesized in mammals. Synthetic glyceryl triheptanoate was selected as an unnatural free fatty acid source, as *n*-heptanoic acid (C7:0), a hydrolyzed from of glyceryl triheptanoate, does not naturally exist in mammals. Moreover, *n*-heptanoyl ghrelin seems to be easily separated from natural ghrelin by HPLC. The results are shown in Fig. 6.

Stomach extracts of glyceryl triheptanoate-treated mice were fractionated by HPLC (upper panel). The concentration of ghrelin in each fraction (from 0.2 mg stomach tissue equivalent) was monitored by C-RIA (middle panel) and N-RIA (lower panel). Ghrelin immunoreactivities, represented by solid bars, were separated into three major peaks (peaks a, b, and c) by C-RIA (middle panel) and two major peaks (peaks d and e) by N-RIA. Peaks b and d were observed only after ingestion of glyceryl triheptanoate.

Of the several immunoreactive peaks detected, the retention times of the isolated ghrelin peptides, peaks a and c, corresponded to des-acyl ghrelin and *n*-octanoyl ghrelin, respectively (Fig. 6). Peak b ghrelin immunoreactivity was observed only in mice fed glyceryl triheptanoate and could not observed in mice fed any other free fatty acids or triglycerides examined, including *n*-hexanoic acid, *n*-octanoic acid, *n*-lauric acid, *n*-palmitic acid, or the corresponding triglyceride forms. The retention time of peak b was between that of *n*-hexanoyl and *n*-octanoyl ghrelins.

HPLC fractionation measured by ghrelin N-RIA exhibited two acyl-modified ghrelin immunoreactivities, found in peaks d and e. From the retention time of synthetic *n*-octanoyl ghrelin by HPLC, peak e corresponded to *n*-octanoyl ghrelin. The retention time of peak d is identical to that of peak b from the C-RIA analysis above. The concentration of peak d measured by N-RIA (74.9 fmol/tube) was lower than the concentration expected from peak b determined by C-RIA (466.3 fmol/tube). These data indicate that the immunoreactive ghrelin in peak d (and peak b) was not modified with an *n*-octanoyl group. Based on the findings above, peaks b and d immunoreactivity is likely *n*-heptanoyl ghrelin. The same peaks (peak b and d) of ghrelin immunoreactivity were also detected from the stomach extracts of mice fed *n*-heptanoic acid (data not shown).

### 7) Purification of n-heptanoyl ghrelin

To confirm that the ingested glyceryl triheptanoate is directly used for the *n*-heptanoyl modification of ghrelin, acyl-modified ghrelins were purified from the stomach tissues of mice fed glyceryl triheptanoate-containing food for 4 days. The sample eluted from an anti-rat ghrelin immunoaffinity column was subjected to HPLC. The results are shown in Fig. 7.

Peak a was observed only in samples from glyceryl triheptanoate-treated mice. Based on the retention times of HPLC and MALDI-TOF-MS analysis, peak b corresponded to *n*-octanoyl ghrelin. Arrows indicate the positions of elution of *n*-hexanoyl (I), *n-*octanoyl (II), and *n*-decanoyl (III) ghrelin, respectively.

From the results of the final purification of ghrelin peptides from the stomachs of treated mice, peak b in Fig. 7 was identified as *n-*octanoyl ghrelin based on its retention time by HPLC. An extra peak eluting at a retention time of 18.4 minutes (peak a in Fig. 7) was observed only after ingestion of glyceryl triheptanoate. This peak eluted at a retention time between that of *n*-hexanoyl- and *n*-octanoyl ghrelins. This peak a peptide was purified and subjected to amino-acid sequence analysis and mass spectrometry.

The purified peptide obtained from HPLC peak a (Fig. 7) was composed of 28 amino acids with an identical amino-acid sequence to that of mouse ghrelin. Ghrelin-like peptide purified from Fig. 7 peak a was subjected to matrix-assisted laser desorption/ionization time-of-flight mass spectrum. The results are shown in Fig. 8.

The results show that the mass ranges from 3131.0 to 3477.0 (m/z). From the averaged 100 mass spectra acquired in positive ion mode (average [M+H]+ : 3301.9), the molecular weight of the peak a peptide was calculated to be 3300.9. B, Structure of *n*-heptanoyl (C7:0) ghrelin. The calculated molecular weight of *n*-heptanoyl ghrelin is 3300.86.

B represents the structure of *n*-heptanoyl (C7:0) ghrelin. The estimated molecular weight of the peptide calculated from m/z value of MALDI-TOF-MS was 3300.9. Modification of ghrelin with an *n-*heptanoyl group at the Ser³ residue would produce a theoretical molecular weight of approximately 3300.86 (Fig. 8B). This is nearly the same as the molecular weight measured by MALDI-TOF-MS. Thus, the purified peptide in peak a was concluded to be *n*-heptanoyl ghrelin. No additional peak was observed in the final purification step, indicating that the *n*-heptanoyl group hydrolyzed from the ingested glyceryl triheptanoate could be directly transferred to the Ser³ residue of ghrelin.

### 8) Molecular forms of circulating ghrelin peptides after glyceryl triheptanoate ingestion

To examine whether unnatural *n*-heptanoyl ghrelin synthesized in mice stomach after glyceryl triheptanoate ingestion is secreted into the circulation, the molecular forms of acyl-modified ghrelins from the plasma of mice fed glyceryl triheptanoate-containing food for 4 days were determined. Plasma samples collected from control mice fed standard chow (A) and glyceryl triheptanoate-treated mice (B) were fractionated by HPLC and measured for ghrelin immunoreactivity by C-RIA. The results are shown in Figure 9.

Arrows indicate the elution positions of des-acyl ghrelin (I) and *n*-octanoyl ghrelin (II). Solid bars represent plasma ghrelin immunoreactivities.

Plasma ghrelin immunoreactivities in control mice were separated into two major peaks (peaks a and b in Fig. 9A) and a minor peak (peak c in Fig. 9A). Plasma ghrelin immunoreactivities in glyceryl triheptanoate-treated mice were separated into two major peaks (peaks d and e in Fig. 9B) and two minor peaks (peaks f and g in Fig. 9B).

In the figure, peaks b and e corresponded to des-acyl ghrelin and peaks c and g corresponded to *n*-octanoyl ghrelin. The newly appeared peak f showed the same retention time with that of *n-*heptanoyl ghrelin purified from stomach in mice after treatment with glyceryl triheptanoate.

Peaks a and d are thought to be C-terminal portion of ghrelin peptide resulted from protease digestion, however, the exact molecular form is not yet determined.

Peak f, eluted at 18.0-18.5 min, was observed only in samples from glyceryl triheptanoate-treated mice. This analysis revealed the existence of plasma ghrelin molecule which had the same retention time with that of *n*-heptanoyl ghrelin purified from stomach in glyceryl triheptanoate fed mice (peak f in Fig. 9B). These results indicate that although *n*-heptanoyl ghrelin is an unnatural form of ghrelin artificially synthesized *in vivo* by glyceryl triheptanoate ingestion, it is definitely released into the circulation.

### 9) Activity of n-heptanoyl ghrelin

Using the ghrelin calcium-mobilization assay, the activity of *n*-heptanoyl ghrelin to stimulate GHS-R (ghrelin receptor) activity was examined.

Time courses of fluorescence changes induced by *n*-octanoyl ghrelin (closed circle), *n*-heptanoyl ghrelin (open circle), and *n-*hexanoyl ghrelin (closed triangle) in GHS-R-expressing cells are shown. Peptides (1 x 10⁻⁸ M) were added at the time indicated by the arrow. The results are shown in Fig. 10

*n*-Heptanoyl ghrelin induced [Ca²⁺]i increases in GHS-R-expressing cells; the time course of these [Ca²⁺]i changes were similar to those induced by *n*-octanoyl ghrelin (Fig. 10). While the agonistic activity of *n*-heptanoyl ghrelin for GHS-R calculated from area under the curve (AUC) of the response curve was approximately 60% that of *n*-octanoyl ghrelin, the value was three times higher than that of *n-*hexanoyl ghrelin (Fig. 10). Thus, *n*-heptanoyl ghrelin possesses GHS-R-stimulating activity.

### (3) Mechanism of Acylation

As a result of experiments, it was revealed that ingested medium-chain fatty acids (MCFAs) and medium-chain triacylglycerides (MCTs) stimulate acyl-modification of ghrelin without increasing total ghrelin mRNA expression and peptide levels. This indicates that these exogenous MCFAs and MCTs are used directly for acyl modification of ghrelin. Further, ingestion of synthetic *n*-heptanoic acid led to the production of an unnatural *n*-heptanoyl ghrelin *in vivo.* These results support that absorbed MCFAs can serve as a direct source for fatty acids in the acyl modifications of ghrelin.

The present invention also opens the way for identification of the molecular mechanism of ghrelin acyl-modification and the enzyme responsible for this modification. The experimental results indicate that ghrelin Ser *O*-acyltransferase that functions in mice likely catalyzes the acyl-modification of *n*-hexanoyl, *n*-heptanoyl, *n*-octanoyl, and *n-*decanoyl ghrelins. Such an enzyme did not catalyze acyl-modification of ghrelin after ingestion of glyceryl tripalmitate, long-chain triacylglycerides (LCTs), and glyceryl tributyrate, a short-chain triacylglycerides (SCTs). These results indicate that the enzyme catalyzing the acyl-modification of ghrelin in mice is a medium-chain (C6:0 to C10:0) acyltransferase with a preference for MCTs (MCFAs) in the acyl-modification of ghrelin.

As most acyltransferases use acyl-CoA as a source of lipid for acyl-modification [D27, D28], the present invention shows the possibility that a portion of MCFs either orally ingested or metabolically reproduced is converted into medium-chain acyl-CoA and used for acyl modification of ghrelin.

### Example 2: Tests in Human

### (1) Effect of Administration of Medium-Chain Fatty acid Triglyceride (MCT) on Human Modified Ghrelin Concentration

### 1) Materials and methods

Racol (Otsuka Pharmaceutical Co., Ltd.) was used as an enteral nutrition agent. The tricaprilin content in Racol is 1500 mg/200 ml. Tricaprin is a medium-chain fatty acid triglyceride (MCT) composed of glycerin and octanoic acid (carbon number (C) 8).

Racol was administered to a low body weight infant (BMI 10) for 18 days at a daily dose of 900 ml (6.75 g as tricaprilin). The modified ghrelin (acylated ghrelin) content in the blood was measured before and 18 days after the administration. The modified ghrelin content in the blood were measured by C-RIA and N-RIA in the same manner as those in Example 1.

### 2) Results

The results of measurement of modified ghrelin (acylated ghrelin) content (fmol/mL) in the blood are shown in Table 2. As is clear from Table 2, when an enteral nutrition agent containing 6.75 g of MCT was administered to a low body weight infant for 18 days, the modified ghrelin content in the blood increased.

One of physiological functions of activated modified ghrelin is release of growth hormones. Growth hormones deeply participate in cell proliferation/division, growth of living body, metabolism of tissues, and the like. The results suggest that administration of MCT is effective for promoting growth of a low body weight infant.

**Table 2**

| | Acyl ghrelin | acyl ghrelin/des-acyl ghrelin |
|---|---|---|
| Day 0 | 22.24 | 0.112 |
| Day 18 | 65.8 | 0.328 |

### (2) Effect of Ingestion of Edible Fat and Oil Containing Medium-Chain Fatty Acid Triglyceride (MCT) on Muscle Strength

### 1) Materials and methods

Actor M-2 (Riken Vitamin Co., Ltd.) was used as an edible fat and oil. The content of medium-chain fatty acid triglyceride (MCT) (octanoic acid (carbon number (C) 8)) in Actor M-2 is 99.99%.

Healthy adults (n=5, male or female) were made to ingest 5 g each of "Actor M-2" twice a day (total 10g) at the time of breakfast and supper. Muscle strength was evaluated before, and 7 and 14 days after the ingestion. Examination of muscle strength was carried out by measuring the grip strength of the dominant hand using a hand dynamometer (Kabushiki Kaisya Tanita). The measurement was performed twice at an interval of about one minute, and the average value was made as the individual value.

### 2) Results (n = 5, mean ± S.D.)

The results are shown in Fig. 11. It is evident from Fig. 11 that the grip strength (muscle strength) was increased by taking edible fat and oil containing MCT enriched with octanoic acid, without loading a special exercise training.

### (3) Effect of Ingestion of Edible Fat and Oil containing Medium-Chain Fatty Acid Triglyceride on Skin

### 1) Materials and methods

Actor M-2 (Riken Vitamin Co., Ltd.) was used as an edible fat and oil. The content of medium-chain fatty acid triglyceride (MCT) (octanoic acid (carbon number (C) 8)) in Actor M-2 is 99.99%.

Healthy adults (n=5, male or female) were made to ingest 5 g each of "Actor M-2" twice a day (total 10g) at the time of breakfast and supper. Skin barrier function was evaluated on the face skin (right and left cheeks) and the inner arm skin before, and 7 and 14 days after the ingestion. Examination of skin barrier function was carried out by measuring the water content and the water transpiration rate under the conditions of 21°C and 60% RH. The water content was measured with a Corneometer CM825 (Courage + Khazaka Electronic GmbH Inc.), and the water transpiration rate with a Tewameter TM210 (Courage + Khazaka Electronic GmbH Inc.). As for the face skin, measurement was performed on five adjacent spots and the average value was obtained as the individual value. As for the inner arm skin, measurement was performed on two adjacent spots on the inner side of left upper arm and the average value was obtained as the individual value.

### 2) Results (n = 5, mean ± S.D.)

The results obtained in the measurement on the face skin are shown in Figs. 12-15, and those obtained in the measuring on the inner arm skin in Fig. 17. It is evident that the water transpiration rate was decreased and the water content on the skin was increased by ingesting edible fat and oil containing MCT enriched with octanoic acid. These results indicate that the skin barrier function was improved and water transpiration was suppressed so that skin maintains necessary water content.

### LIST OF REFERENCES

1. Kojima M, Hosoda H, Date Y, Nakazato M, Matsuo H, Kangawa K. Ghrelin is a growth-hormone-releasing acylated peptide from stomach. Nature 1999; 402 (6762): 656-60.
2. Hosoda H, Kojima M, Matsuo H, Kangawa K. Ghrelin and des-acyl ghrelin: two major forms of rat ghrelin peptide in gastrointestinal tissue. Biochem Biophys Res Commun 2000; 279 (3): 909-13.
3. Date Y, Kojima M, Hosoda H, Sawaguchi A, Mondal MS, Suganuma T, Matsukura S, Kangawa K, Nakazato M. Ghrelin, a novel growth hormone-releasing acylated peptide, is synthesized in a distinct endocrine cell type in the gastrointestinal tracts of rats and humans. Endocrinology 2000; 141 (11): 4255-61.
4. Date Y, Nakazato M, Hashiguchi S, Dezaki K, Mondal MS, Hosoda H, Kojima M, Kangawa K, Arima T, Matsuo H, Yada T, Matsukura S. Ghrelin is present in pancreatic alpha-cells of humans and rats and stimulates insulin secretion. Diabetes 2002; 51 (1): 124-9.
5. Mori K, Yoshimoto A, Takaya K, Hosoda K, Ariyasu H, Yahata K, Mukoyama M, Sugawara A, Hosoda H, Kojima M, Kangawa K, Nakao K. Kidney produces a novel acylated peptide, ghrelin. FEBS Lett 2000; 486 (3): 213-6.
6. Galas L, Chartrel N, Kojima M, Kangawa K, Vaudry H. Immunohistochemical localization and biochemical characterization of ghrelin in the brain and stomach of the frog Rana esculenta. J Comp Neurol 2002; 450 (1): 34-44.
7. Gnanapavan S, Kola B, Bustin SA, Morris DG, McGee P, Fairclough P, Bhattacharya S, Carpenter R, Grossman AB, Korbonits M. The tissue distribution of the mRNA of ghrelin and subtypes of its receptor, GHS-R, in humans. J Clin Endocrinol Metab 2002; 87 (6): 2988.
8. Arvat E, Di Vito L, Broglio F, Papotti M, Muccioli G, Dieguez C, Casanueva FF, Deghenghi R, Camanni F, Ghigo E. Preliminary evidence that Ghrelin, the natural GH secretagogue (GHS)-receptor ligand, strongly stimulates GH secretion in humans. J Endocrinol Invest 2000; 23 (8): 493-5.
9. Peino R, Baldelli R, Rodriguez-Garcia J, Rodriguez-Segade S, Kojima M, Kangawa K, Arvat E, Ghigo E, Dieguez C, Casanueva FF. Ghrelin-induced growth hormone secretion in humans. Eur J Endocrinol 2000; 143 (6): R11-4.
10. Takaya K, Ariyasu H, Kanamoto N, Iwakura H, Yoshimoto A, Harada M, Mori K, Komatsu Y, Usui T, Shimatsu A, Ogawa Y, Hosoda K, Akamizu T, Kojima M, Kangawa K, Nakao K. Ghrelin strongly stimulates growth hormone release in humans. J Clin Endocrinol Metab 2000; 85 (12): 4908-11.
11. Nakazato M, Murakami N, Date Y, Kojima M, Matsuo H, Kangawa K, Matsukura S. A role for ghrelin in the central regulation of feeding. Nature 2001; 409 (6817): 194-8.
12. Shintani M, Ogawa Y, Ebihara K, Aizawa-Abe M, Miyanaga F, Takaya K, Hayashi T, Inoue G, Hosoda K, Kojima M, Kangawa K, Nakao K. Ghrelin, an endogenous growth hormone secretagogue, is a novel orexigenic peptide that antagonizes leptin action through the activation of hypothalamic neuropeptide Y/Y1 receptor pathway. Diabetes 2001; 50 (2): 227-32.
13. Tschop M, Smiley DL, Heiman ML. Ghrelin induces adiposity in rodents. Nature 2000; 407 (6806): 908-13.
14. Wren AM, Small CJ, Abbott CR, Dhillo WS, Seal LJ, Cohen MA, Batterham RL, Taheri S, Stanley SA, Ghatei MA, Bloom SR. Ghrelin causes hyperphagia and obesity in rats. Diabetes 2001; 50 (11): 2540-7.
15. Nagaya N, Uematsu M, Kojima M, Ikeda Y, Yoshihara F, Shimizu W, Hosoda H, Hirota Y, Ishida H, Mori H, Kangawa K. Chronic administration of ghrelin improves left ventricular dysfunction and attenuates development of cardiac cachexia in rats with heart failure. Circulation 2001; 104 (12): 1430-5.
16. Nagaya N, Kangawa K. Ghrelin improves left ventricular dysfunction and cardiac cachexia in heart failure. Curr Opin Pharmacol 2003; 3 (2): 146-51.
17. Enomoto M, Nagaya N, Uematsu M, Okumura H, Nakagawa E, Ono F, Hosoda H, Oya H, Kojima M, Kanmatsuse K, Kangawa K. Cardiovascular and hormonal effects of subcutaneous administration of ghrelin, a novel growth hormone-releasing peptide, in healthy humans. Clin Sci (Lond) 2003; 105 (4): 431-5.
18. Masuda Y, Tanaka T, Inomata N, Ohnuma N, Tanaka S, Itoh Z, Hosoda H, Kojima M, Kangawa K. Ghrelin stimulates gastric acid secretion and motility in rats. Biochem Biophys Res Commun 2000; 276 (3): 905-8.
19. Hosoda H, Kojima M, Matsuo H, Kangawa K. Purification and characterization of rat des-Gln 14-Ghrelin, a second endogenous ligand for the growth hormone secretagogue receptor. J Biol Chem 2000; 275 (29): 21995-2000.
20. Hosoda H, Kojima M, Mizushima T, Shimizu S, Kangawa K. Structural divergence of human ghrelin. Identification of multiple ghrelin-derived molecules produced by post-translational processing. J Biol Chem 2003; 278(1): 64-70.
21. Kaiya H, Kojima M, Hosoda H, Koda A, Yamamoto K, Kitajima Y, Matsumoto M, Minamitake Y, Kikuyama S, Kangawa K. Bullfrog ghrelin is modified by n-octanoic acid at its third threonine residue. J Biol Chem 2001; 276 (44): 40441-8.
22. Kaiya H, Van Der Geyten S, Kojima M, Hosoda H, Kitajima Y, Matsumoto M, Geelissen S, Darras VM, Kangawa K. Chicken ghrelin: purification, cDNA cloning, and biological activity. Endocrinology 2002; 143 (9): 3454-63 .
23. Matsumoto M, Hosoda H, Kitajima Y, Morozumi N, Minamitake Y, Tanaka S, Matsuo H, Kojima M, Hayashi Y, Kangawa K. Structure-activity relationship of ghrelin: pharmacological study of ghrelin peptides. Biochem Biophys Res Commun 2001; 287 (1): 142-6.
24. Chomczynski P, Sacchi N. Single-step method of RNA isolation by acid guanidinium thiocyanate-phenol-chloroform extraction. Anal Biochem 1987; 162 (1): 156-9.
25. Hillenkamp F, Karas M. Mass spectrometry of peptides and proteins by matrix-assisted ultraviolet laser desorption/ionization. Methods Enzymol 1990; 193: 280-95.
26. Greenberger NJ, Skillman TG. Medium-chain triglycerides. N Engl J Med 1969; 280 (19): 1045-58.
27. Coleman RA, Lewin TM, Muoio DM. Physiological and nutritional regulation of enzymes of triacylglycerol synthesis. Annu Rev Nutr 2000; 20: 77-103.
28. Eaton S, Bartlett K, Pourfarzam M. Mammalian mitochondrial beta-oxidation. Biochem J 1996; 320 (Pt 2): 345-57.

## Claims

1. A regulator for regulating physiological functions of ghrelin, which comprises a fatty acid of carbon number 2 to 35 or its derivative.

2. The regulator according to claim 1, wherein the physiological function of ghrelin is activity of increasing intracellular calcium ion concentration, activity of promoting growth hormone secretion, activity of promoting eating, regulatory activity relating to fat accumulation, activity of ameliorating cardiac function or activity of stimulating gastric acid secretion.

3. A pharmaceutical composition comprising a regulator set forth in claim 1 or 2.

4. A functional food comprising a regulator set forth in claim 1 or 2.

5. An accelerator for the formation of activated ghrelin, which comprises at least one medium-chain fatty acid of 6 to 12 carbon number or its derivative.

6. The accelerator for the formation of activated ghrelin according to claim 5, which comprises at least one medium-chain fatty acid of 8 to 10 carbon number or its derivative.

7. A functional food containing an accelerator for the formation of activated ghrelin set forth in claim 5 or 6.

8. A composition **characterized in that** it contains a medium-chain fatty acid of 6 to 12 carbon number or its derivative and that it has muscle strengthening activity.

9. A composition comprising a medium-chain fatty acid of 6 to 12 carbon number or its derivative which has skin-beautification activity.

10. A method for preventing or treating disorders associated with physiological functions of ghrelin, which comprises administering an effective amount of a regulator set forth in claim 1, a pharmaceutical composition set forth in claim 3 or a functional food set forth in claim 4 to a subject in need thereof.
